# EUROPEAN PATENT SPECIFICATION

(11) **EP 0 192 671 B1**
(45) Date of publication and mention of the grant of the patent: **29.12.1993**
(21) Application number: 85904069.3
(22) Date of filing: 13.08.1985
(51) Int. Cl.: C07K 15/06, C12P 21/00, G01N 33/574, G01N 33/68

(54) **MINACTIVIN**
MINAKTIVIN
MINACTIVIN

(30) Priority: 13.08.1984 AU 6531/84
(43) Date of publication of application: 03.09.1986
(73) Proprietor: BIOTECHNOLOGY AUSTRALIA PTY. LTD., East Roseville, NSW 2069 (AU); THE AUSTRALIAN NATIONAL UNIVERSITY, Acton, Australian Capital Territory 2601 (AU)
(72) Inventor: STEPHENS, Ross, Wentworth, Holder, ACT 2616 (AU); GOLDER, Jeffrey, Philip, Newport, NSW 2106 (AU); GOSS, Neil, Howard, Wahroonga, NSW 2076 (AU); ANTALIS, Toni, Marie, Drummoyne, NSW 2047 (AU)
(74) Representative: Sheard, Andrew Gregory
(86) International application number: AU8500191
(87) International publication number: WO8601212

(56) References cited:
- EUROPEAN JOURNAL OF BIOCHEMISTRY, vol. 136, 1983, Heidelberg(DE); J.P. GOLDER et al., pp. 517-522#
- CHEMICAL ABSTRACTS, vol. 96, no. 19, 10 May 1982, Columbus, OH (US); H.A. CHAPMAN et al., no. 160042a#
- BIOLOGICAL ABSTRACTS, vol. 78, no. 11, 1984, Philadelphia, PA (US); J.-D. VASSALLI et al., p. 9103, no. 81039#
- CHEMICAL ABSTRACTS, vol. 96, no. 7, 15 February 1982, Columbus, OH (US); V. KLIMETZEK, p. 471, no. 50530a#
- BIOLOGICAL ABSTRACTS, vol. 79, no. 8, Philadelphia, PA (US); O. SAKSELA et al., p. AB-67, no. 64782#

## Description

The present invention relates to a new protein isolated from certain cells, which protein acts as a specific inactivator of urokinase - type plasminogen activator.

This invention relates to specific inactivator of urokinase-type plasminogen activator which has been identified and isolated in adherent monolayer cultures of human blood monocytes, certain macrophages and transformed cells of monocytic origin. The inactivator has been given the name "minactivin", and this name will be used throughout this specification to refer to this product.

Plasminogen activators are proteolytic enzymes present in many animal tissues and secretions. The most widely known function of plasminogen activator is to catalyse the conversion of plasminogen to its active form, plasmin. A major role of plasmin is that of fibrinolysis, or the dissolution of blood clots. Two types of mammalian plasminogen activator, differing in molecular weight, amino acid sequence and immunological reactivity have been characterised in humans; urokinase-type plasminogen activator and tissue-type plasminogen activator. Several major types of human cancers, including carcinomas of the breast, lung, colon and prostate have been shown to produce abnormally high levels of the urokinase-type plasminogen activator, and several lines of evidence indicate that this factor is involved in the process by which tumours invade surrounding tissues.

### GENERAL DESCRIPTION OF THE INVENTION

The present invention is based upon the discovery that the culture supernatant from adherent monolayer cultures of human blood monocytes contains minactivin [Golder & Stephens, Eur. J. Biochem., 136 (1983), 517-522]. Early work showed that cultured human monocytes concomitantly secrete pro-urokinase and a plasminogen activator specific inhibitor [Vassalli et al, J. Exp. Med. (1984) 159, 1653-1668]. This molecule was not well characterised by either Golder and Stephens or Vassalli et al and there are discrepancies between the results they report. The molecule has been characterised by Vassalli et al as having a molecular weight of approximately 39 000 by its ability to increase the relative molecular weight of labelled urokinase on SDS-PAGE whilst Golder and Stephens by gel filtration assign a molecular weight of 60 000 - 70 000. Vassalli's evidence for the existence of minactivin is indirect and does not show that a specific entity from culture supernatant was associated with that shift.

Potential of the molecule for diagnostic and therapeutic use was suggested by this early work. Minactivin is a potent inactivator or urokinase-type plasminogen activator. Minactivin has been shown to be a natural inactivator of this plasminogen activator which is associated with invasive tumours, and is therefore indicated to be a crucial element in the body's normal defence against tumour invasion and metastasis. Minactivin furthermore has a range of potential applications as a clinical reagent in the area of human medicine, in the in vitro and in vivo diagnosis of various types of carcinomas, arthritis and fibrinolysis as well as in the treatment of these conditions. Minactivin has also been isolated and identified from cultures of macrophages and transformed cells of monocytic origin.

According to a first aspect of the present invention, there is provided the protein minactivin:
(a) having an apparent molecular size of 60,000-70,000 by gel filtration chromatography, identical to a serum albumin standard;
(b) capable of specifically inhibiting human urokinase-type plasminogen activator but not tissue-type plasminogen activator in the presence of fibrin;
(c) being heat labile at temperatures above 56°C;
(d) being stable to freezing at -20°C and thawing;
(e) being stable in a pH range of 5 to 9 at 4°C;
(f) having a pI between 5.0 and 7.8;
(g) being capable of forming a detergent resistant complex with human urokinase or human urokinase-type plasminogen activator;
(h) producing at least one band with a Mᵣ of 32kD, 39-48kD or 60kD on non-reducing SDS-PAGE which band(s) disappear(s) when the protein is incubated with urokinase; and
(i) being purified to a specific activity of at least about 8,900 U/mg.

The minactivin of the invention is preferably purified to the order of about 8,900 U/mg.

In another aspect of the present invention, there is provided a process for the preparation of minactivin as defined above, which process comprises stimulating cells producing minactivin, the cells being cultured in vitro; and recovering minactivin from the culture supernatant by hydrophobic interaction chromatography followed by affinity chromatography or preparative non-denaturing gel electrophoresis. The cells which may be cultured to produce minactivin include human blood monocytes, minactivin-producing macrophages or transformed cells of monocytic origin.

The in vitro stimulation of cells to produce minactivin can be achieved using bacterial lipopolysaccharide, dexamethasone or a phorbol ester. Culture supernatant containing minactivin secreted by the cells can be treated so as to concentrate the minactivin contained therein, and then treated so as to produce minactivin in an at least partially purified form, from which other components of the culture supernatant have been removed. This concentration and purification may be achieved, for example, by hydrophobic interaction chromatography on phenyl-Sepharose, or ion exchange chromatography using DEAE-Sepharose, followed by elution of the desired product therefrom. This product may be further purified by affinity chromatography, for example, on Cibacron Blue-Sepharose or hydroxylapatite to remove further impurities from the minactivin-containing product.

Investigations have shown that minactivin is a protein characterized by an apparent molecular size of approximately 60-70,000 (by gel filtration chromatography), and that its inhibitory effect is specific for plasminogen activators (such as urokinase) of Mᵣ 52,000 (HPA52) and 36,000 (HPA36) as shown by (i) inhibition of plasminogen-dependent fibrinolysis, (ii) inhibition at the level of plasminogen activation in a colorimetric assay, and (iii) irreversible loss of plasminogen activating activity, as evidenced by electrphoresis, after preincubation with culture media. Complex formation by minactivin and the HPA36 form of plasminogen activator was shown to be detergent resistant and characterized by a Mᵣ in the range of 70-75,000 (minactivin-HPA36 complex).

It has further been shown that minactivin preferentially inhibits urokinase-type plasminogen activator. and not trypsin, plasmin or "tissue" type plasminogen activator (HPA66 or 66,000 Mᵣ activator).

Minactivin has been found to be produced not only by human monocytes, but also by certain macrophages and transformed cells of macrophage lineage. In particular, peritoneal macrophages and bone marrow macrophages, when cultured in adherent monolayer cultures, have been found to produce and secrete appreciable amounts of minactivin. The human macrophage cell line, U937, has also been found to produce minactivin when cultured in the presence of dexamethasone. In a further development of this aspect of the invention, it has been discovered that the production of minactivin by blood monocytes in adherent monolayer culture can be stimulated or enhanced by the activation of the cells by use of muramyl dipeptide (adjuvant peptide), phorbol esters such as phorbol myristate acetate bacterial lipopolysaccharide or the lymphokine-containing supernatants from cultures of activated human lymphocytes.

When added to cultures of the human colon carcinoma cell line. COLO394, minactivin was found to specifically inactivate plasminogen activator (HPA52) as evidenced by colorimetric assay and electrophoresis. Furthermore, minactivin was capable of specifically inhibiting plasminogen activator activity in human tumour tissue homogenates obtained following surgical resection. It has been demonstrated that plasminogen activator levels are considerably elevated in tumour tissues compared with normal tissues from corresponding colons. Similar observations have been documented by other investigators with respect to a wide range of solid tumours.

Therefore, in view of minactivin's specificity for urokinase-type plasminogen activator and the markedly enhanced levels of this plasminogen activator in tumour tissues, this product has application as a reagent for locating and defining the boundaries of tumours in histological specimens and in vivo. Thus, minactivin can be used to image tumours in vivo. In particular, solid tumours such as breast, prostate, colon and lung, which produce plasminogen activator, can be targeted by minactivin. While such cancers are generally redressed by surgical intervention, the use of minactivin in imaging such tumours can be of particular benefit in the identification of small metastatic cancers arising after surgical intervention. It will, of course, be appreciated that since minactivin is a natural product, it has the advantage of a lack of antigenicity and the avoidance of clearance problems within the body. In imaging such tumours in vivo, minactivin may be used in a labelled from, for example, labelled with an appropriate radioactive isotope (such as technetium⁹⁹), and after administration the location and boundaries of tumours imaged by minactivin may be determined by known radioisotopic procedures.

In the location and definition of boundaries of tumours in histological specimens, minactivin can be conjugated either with a radioactive isotope as described above, or it may be labelled by conjugation, using standard procedures, with an appropriate enzyme (or other suitable reagent). On contacting a histological specimen with a minactivin-enzyme complex, the minactivin will react with the urokinase at its place of secretion and high concentration, thereby identifying the tumour boundaries. Detection of the minactivin-enzyme complex found at these locations of high concentration can be effected by known means, for example, by applying a substrate for the enzyme, followed by an indicator to detect action of the enzyme on the substrate.

In addition to the imaging of tumours, minactivin is also indicated for use in direct treatment of tumours by decreasing tumour plasminogen activator activity and, since this activity is implicated in the process by which the tumours invade surrounding tissues, regulation and particularly inhibition of tumour invasion can be achieved.

### BRIEF DESCRIPTION of the FIGURES

### Figure 1

Production of minactivin by blood monocyte cultures. Adherent monocytes were cultured in: control media △ △ with lipopolysaccharide (0.1 ug.ml) ○ - - - - - - - ○ or with muramyl dipeptide (10 ug/ml) □ ...............□ A. Culture supernatants were harvested, assayed with urokinase and replaced with fresh media every 24 hrs. B. Content of minactivin in cell lysates.

### Figure 2

Dependence of minactivin activity on monocyte cell number in culture. Supernatants from 24 hr cultures with cell numbers shown were assayed undiluted △ △ , diluted 1:5 □ ...............□ , 1:10 ○ - - - - - -○, or 1:20 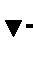 - - - - - - - 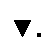 .

### Figure 3

Production and secretion of minactivin by human peritoneal macrophage culture. Adherent macrophages were grown in control media △ △ , or with muramyl dipeptide (10 ug/ml) □ ............... □. The lower two curves represent lysates and the upper two curves, supernatants.

### Figure 4

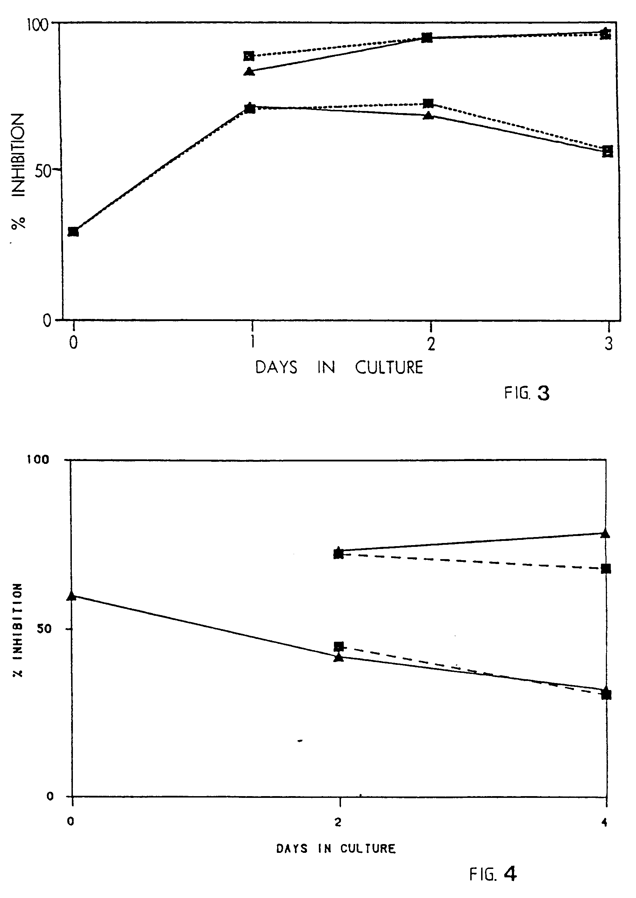

Production and secretion of minactivin by human bone marrow macrophages in secondary culture. Conditions as for Fig. 3.

### Figure 5

Effect of Minactivin on Fibrinolysis Protease solutions (10 ul) were preincubated with (upper two rows) or without (lowest row) minactivin culture supernatant (10 ul) and the mixture (5 ul) applied to wells cut in a plasminogen supplemented fibrin/agarose gel. The enzymes and the amounts used per 20 ul of preincubation were: 2. trypsin, 100 ng; 3. plasmin, 600 ng; 4. CUK, (Calbiochem Urokinase) 50 mPU: 5. SUK. (Sigma Urokinase) 100 mPU: and 6, melanoma culture supernatant undiluted. The control wells 1 and 7 contained assay buffer and minactivin supernatant respectively. The gel was incubated for 20 hrs at 37°.

### Figure 6

SDS-PAGE after Preincubation with Minactivin Plasminogen activators from CUK (30 mPU, lanes 1 and 2), MLA144 (30 ul undiluted culture supernatant, lanes 3 and 4), human melanoma (20 ul culture supernatant, lanes 5 and 6), and rat 13762 tumour (20 ul 1% homogenate, lanes 7 and 8) were preincubated with (even numbers) and without (odd numbers) minactivin culture supernatant (30 ul) before application to SDS-PAGE. The bands were developed in a fibrin overlay gel by incubation for 20 hours at 37°.

### Figure 7

Inactivation of CUK by preincubation with minactivin. Aliquots of urokinase (4 mPU) were preincubated with monocyte culture supernatant (6 ul) at 23° for the times shown, then assayed colorimetrically as described in the test. The upper curve is for urokinase preincubated with buffer.

### Figure 8

Titration of CUK with minactivin. Aliquots of urokinase (4 mPU) were preincubated with dilutions of the minactivin supernatant (20 ul), then assayed colorimetrically as described in the text.

### Figure 9A

Protease inhibitor controls for CUK inactivation by minactivin. Aliquots of urokinase (20 mPU) were preincubated with the following protease inhibitors: lane 1, buffer control; lane 2, Trasylol (0.2 mg/ml); lane 3, alpha₁ antitrypsin (16 ug/ml); lane 4. tranexamic acid (3 mM); lane 5, iodoacetamide (3 mM); lane 6, EDTA (6 mM); lane 7, soybean trypsin inhibitor (0.16 mg/ml); lane 8, SDS (0.6%); lane 9, benzamidine (3 mM); and lane 10, minactivin culture supernatant (20 ul).

### Figure 9B

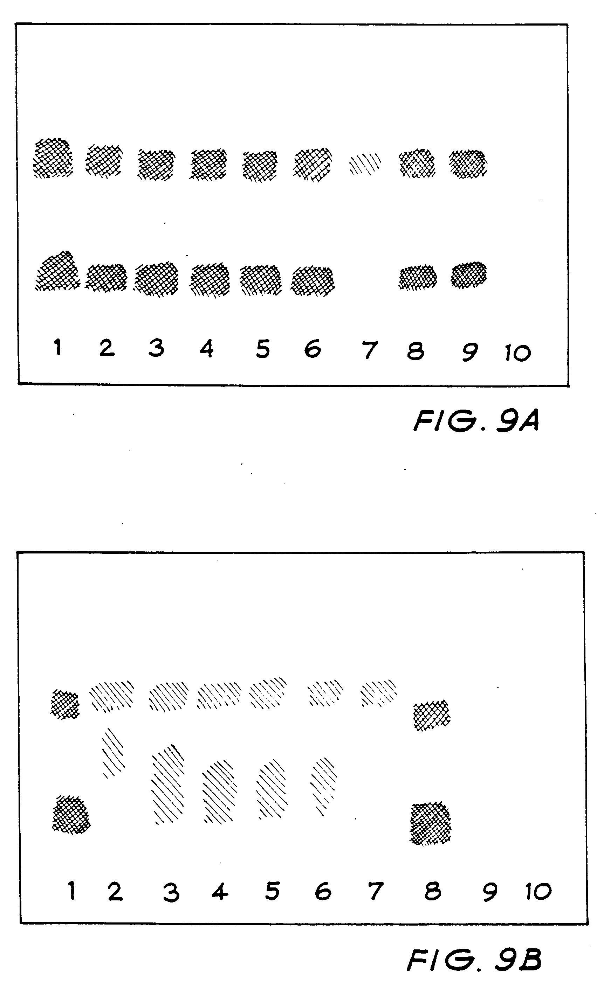

Inactivation of CUK by minactivin in the presence of protease inhibitors. The preincubations in Fig. 9A above were repeated with minactivin culture supernatant (20 ul) included in each protease inhibitor preincubation (lanes 2-9).

### Figure 10

Elution profile of 2.5 ml of minactivin culture supernatant without albumin supplement , and 1.0 ml of unconditioned RPMI with 1% human serum albumin - - - - - - - assayed for urokinase inhibition and protein respectively. The column was packed with Sephacryl S300 (95 x 1.5 cm) and eluted with 50 mM glycine (pH 7.8) containing 0.15 M NaCl at a flow rate of 6 ml/hr. Arrows indicate void volume and bed volume.

### Figure 11

Radial diffusion fibrin gel assays of protease inhibition. Row A, human melanoma cell line supernatant containing HPA66; Row B, urokinase containing HPA52 and HPA36; Row C, trypsin and Row D, plasmin. Columns 1 and 2 included supernatants from cultures of human peritoneal macrophages and blood monocytes respectively, while columns 4-6 included lysates of bone marrow macrophages, peritoneal macrophages and blood monocytges respectively. Columns 3 and 7 represent controls containing culture media and lysis buffer respectively.

### Figure 12

Radial diffusion fibrin gel assays of protease inhibition. Row A, Buffer control; Row B, phenyl-Sepharose purified minactivin from monocytes; Row C, phenyl-Sepharose purified minactivin from U937 cells; Row D, placental inhibitor (Calbiochem). Column 1, porcine plasmin 60 ug/ml (sigma); Column 2, urokinase containing HPA52; CUK, 5 PU/ml; Column 3. urokinase containing HPA36, SUK, 5 PU/ml; Column 4, human melanoma cell line supernatant, MM170, containing HPA66; Column 5, mouse urokinase, 1/10 dilution mouse urine.

### Figure 13

Electrophoretic Mobility of U937 and Monocyte derived Minactivin. Samples were electrophoresed on a 6-16% gradient nondenaturing (no SDS) polyacrylamide gel, incubated with 10 mls SUK 1 PU/ml for 1 hr at 23°C. overlayed on a fibrin/agarose gel, and developed overnight. Identical samples were also electrophoresed and then stained with Coomassie Blue. Lane 4, molecular weight standards; Lane 3, phenyl-Sepharose purified U937 minactivin; Lane 2, phenyl-Sepharose purified monocyte minactivin; Lane 1, as lane 3.

### Figure 14

Purification Profile of Monocyte Minactivin on Phenyl-Sepharose. A column of phenyl-Sepharose (bed volume 110 ml) was first equilibrated with 50 mM glycine buffer pH 7.8 containing 2 M NaCl. Monocyte supernatant adjusted to 2 M NaCl was then applied, followed by washing with additional glycine-NaCl. Elution of bound minactivin occurred during a descending gradient of NaCl in glycine buffer. Traces are: Protein (by Lowry assay. OD750 nm) NaCl concentration ............. and minactivin inhibition of urokinase (inverted peak) - - - - - - .

### Figure 15

In vivo Labelling of Monocyte Minactivin.
Adherent monocytes were cultured in the presence of muramyl dipeptide as described in the text with the addition of ³⁵S-Methionine (1 mCi, 800 Ci/mmol, Amersham). Aliquots of the supernatant were removed at the times indicated over the course of 3 days. Minactivin was then purified from the supernatant using phenyl- Sepharose as described in the text. Each aliquot was concentrated 50 fold using a Centricon 30 (Amicon), and 30 ul analysed by SDS-PAGE using a 5-15% acrylamide gradient. The radiolabelled proteins were detected by fluorography (Amplify, Amersham) and the gel exposed on Kodak O-MAT X-ray film for 10 days. Lanes 2-6 are samples removed at the times indicated, Lane 7 is the phenyl- Sepharose purified minactivin preparation. Lanes 1 and 8 are high and low molecular weight standards respectively.

### Figure 16

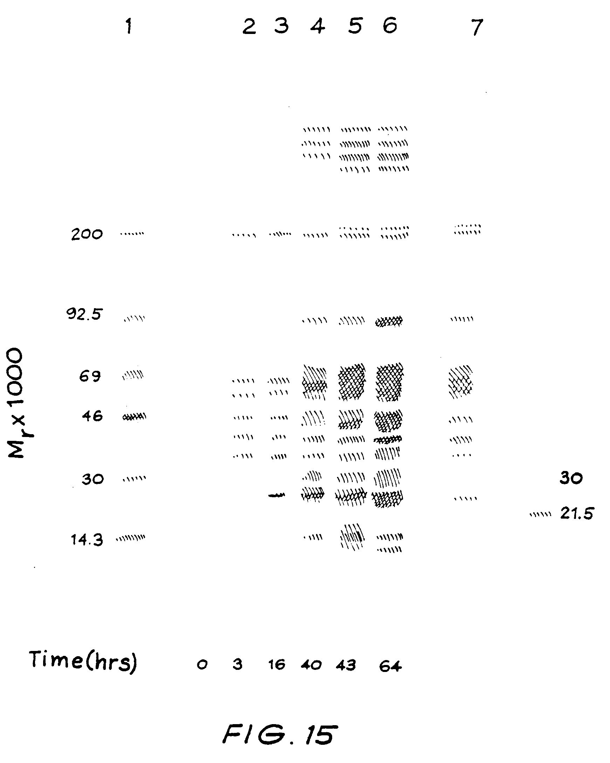

Purification of In Vivo Labelled Monocyte Minactivin and Urokinase Complex Formation.
Conditions essentially as for Fig. 15 and as described in the text. Each sample was concentrated 10 fold before loading 30 ul on the gel. Exposure time was 18 days. Lanes A, phenyl-Sepharose purified minactivin; Lanes B, blue Sepharose purified minactivin; Lanes C, identical to B except 0.1% 2-mercaptoethanol added to the samples immediately prior to electrophoresis. Each sample is shown (-) or (+) preincubation with 27 PU SUK for 90 min at 23°C. Outside lanes are molecular weight standards.

### Figure 17

Effect of Gelatin on Minactivin Production.
U937 cells (1.3 X 10⁶ cells/ml) were cultured in serum- free medium with dexamethasone as described in the text, in the presence (x x) and in the absence (^{.} ^{.}) of 0.7% gelatin as described.

### Figure 18

Phenyl-Sepharose Chromatography using a Step pH Elution.
The chromatography was performed as described in Purification Example 1. At position A the eluent was changed to 50 mM citrate, pH 5.0, 0.5 M NaCl, and at position B the eluent was changed to 50 mM glycine, pH 7.8.

### Figure 19

DEAE-Sepharose Chromatography using Step-wise pH Elution.
Minactivin containing supernatants were fractionated on a DEAE-Sepharose column as described in Purification Example 3. Minactivin activity is shown in the open columns and protein - - - - - A275.

### Figure 20

Preparative Nondenaturing Gel Electrophoresis.
A phenyl-Sepharose purified minactivin preparation was further fractionated on by nondenaturing gel electrophoresis as described in Purification Example 4. Minactivin activity eluted from the gel slices is shown in the open columns. Beneath is shown an equivalent gel lane stained by the silver stain method.

### Figure 21

DEAE-Sephacel Chromatography using Salt Gradient Elution.
Minactivin containing supernatants were fractionated on a DEAE-Sephacel column as described in Purification Example 6. Units of biological activity are shown in the open columns; ......... , A275; - - - - - , NaCl concentration.

### Figure 22

DEAE-Sephacel Chromatography using a pH Gradient.
Minactivin containing supernatants were fractionated as described in Purification Example 7. Units of biological activity are shown in the open columns; protein content is shown in the closed columns; - - - - - - , A275, ― ― ― ― , pH.

### Figure 23

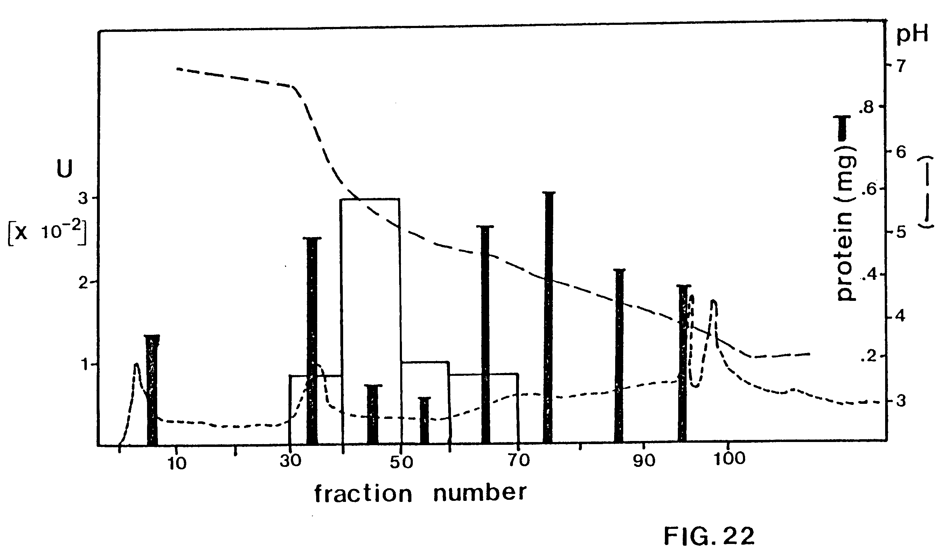

SDS-PAGE of Fractions from DEAE-Sephacel
Aliquots equivalent to 70 ug of protein were removed from pooled fractions of the DEAE-Sephacel chromatography shown in Figure 22 and analysed by SDS-PAGE as described by Laemmli (26). Lane 1, molecular weight markers; lane 4, fractions 30-40; lane 5, fractions 40-50; lane 6, 50-60; lane 7, 60-70; lane 8, 70-80; lane 9, 80-90; lane 10, 90-100.

### Figure 24

Hydroxylapatite Chromatography
Minactivin containing supernatants were fractionated as described in Purification Example 8. Units of biological activity are shown in the open columns; protein content is shown in the closed columns; ........... , A275; - - - - - , sodium phosphate concentration.

### Figure 25

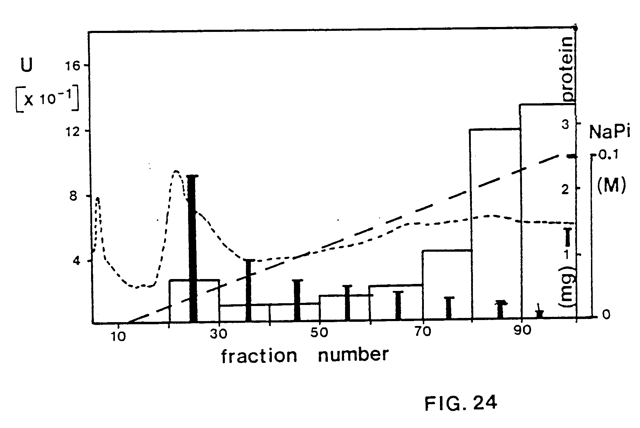

SDS-PAGE of Fractions from Hydroxylapatite Chromatography.
Aliquots equivalent to 70 ug of protein were removed from selected pooled fractions of the hydroxylapatite chromatography shown in Figure 24, and analysed on SDS-PAGE as described by Laemmli (26). Lane 8, fractions 70-80; lane 9, fractions 80-90; lane 10, 90-100.

### Figure 26

Frequency distribution histograms showing the results from colorimetric assays of plasminogen activator in homogenates of human colonic mucosae. The plasminogen substrate contained traces of plasmin, such that results represent the sum of proenzyme and active enzyme. The activity is expressed as the absorbance at 412 nm. Under the same assay conditions, 4 mPU of commercial urokinase produced an absorbance of 0.9 at 412 nm.

### Figure 27

Plasminogen activator activity of 15 colon tumour specimens (striped rectangles) compared to the activity in normal tissue (solid rectangles) from the same colons. The samples are ranked left to right according to the increasing value of the tumour:normal ratio (T/N).

### Figure 28A

Types of plasminogen activator present in typical homogenate of normal colonic mucosa shown by fibrin agarose overlay after SDS-PAGE, Lane 1 represents untreated sample, Lanes 2-4 show the effects of incubating the homogenate for 30, 60 and 120 min at 37° with human plasminogen. Zones of lysis are; a) plasminogen activator of 110,000; b) plasminogen activator of Mᵣ 96,000; c) plasmin, Mᵣ 85,000 and d) HPA66, Mᵣ 66,000.

### Figure 28B

Types of plasminogen activator present in homogenate of a typical colon cancer specimen as shown by fibrin agarose overlay after SDS-PAGE. Lane 1 represents the untreated sample, Lanes 2-4 after 30, 60 and 120 min incubation at 37° with human plasminogen. Zones A-D as for normal colon. Zone E is HPA52 (Mᵣ 52,000) and Zone F is HPA33-35 (Mᵣ 33-36,000).

### Figure 29A

The effect of minactivin on plasminogen activators in a homogenate of a sample histologically normal colon expressing atypical levels of HPA52 activity. Lane 1 shows control treated sample. Lane 2 shows the effect of preincubation for 60 min at 23° with minactivin. Lane 3 represents the effect of preincubation for 30 min at 37° with plasminogen and then for 60 min at 23° with control buffer. In Lane 4 the homogenate was preincubated for 30 min at 37° with plasminogen, and then exposed in minactivin for 60 min at 23°. Zones of lysis are: A - plasmin, Mᵣ 85,000; B - HPA66, Mᵣ 66,000; C - HPA 52, Mᵣ 52,000 and D - HPA33-36, Mᵣ 33-36,000.

### Figure 29B

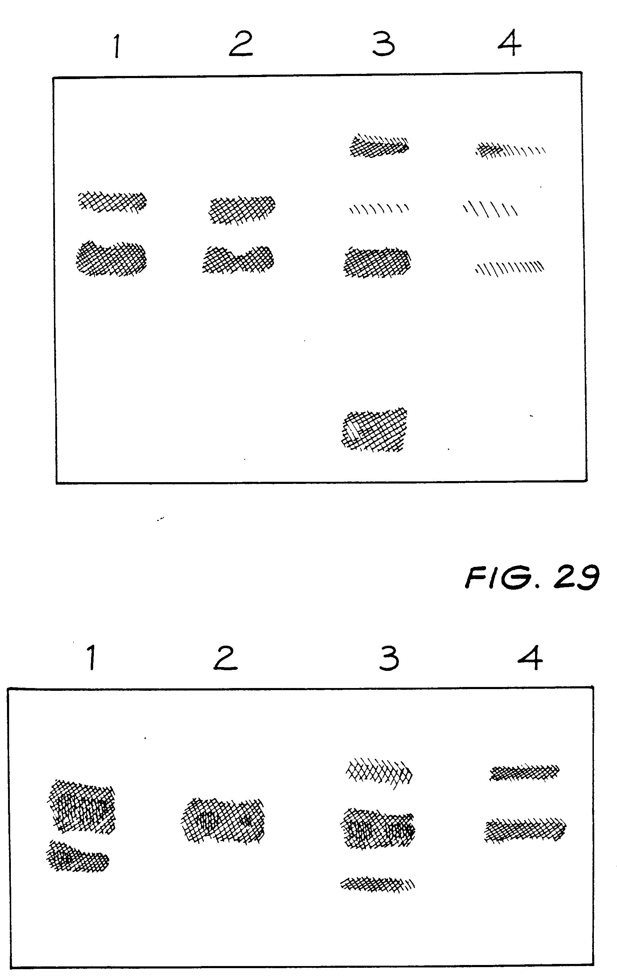

Colon tumour homogenate treated as in Figure 29A.

### Figure 30

Titration of the colorimtetric activity of human urokinase with minactivin. The urokinase was preincubated with minactivin for 90 mins at 23° before dilution to the range suitable for assay. The three curves represent, from left to right, 1000 mPU, 100 mPU and 10 mPU respectively of urokinase.

### Figure 31

Titration of the colorimetric activity of human plasminogen activators from colon cells and tissues with minactivin. The three curves represent, from left to right, an extract of normal mucosa (approx. 0.5 mPU), and
extract of colon tumour (approx. 10 mPU). Note that the latter is later known to be proenzyme.

### Figure 32

Effect of minactivin on tumour cell culture plasminogen activator. Lane 1, culture with no additions; Lane 2, with plasminogen Lane 3, with plasminogen and minactivin; Lane 4, with plasminogen, trasylol and minactivin.

### Figure 33

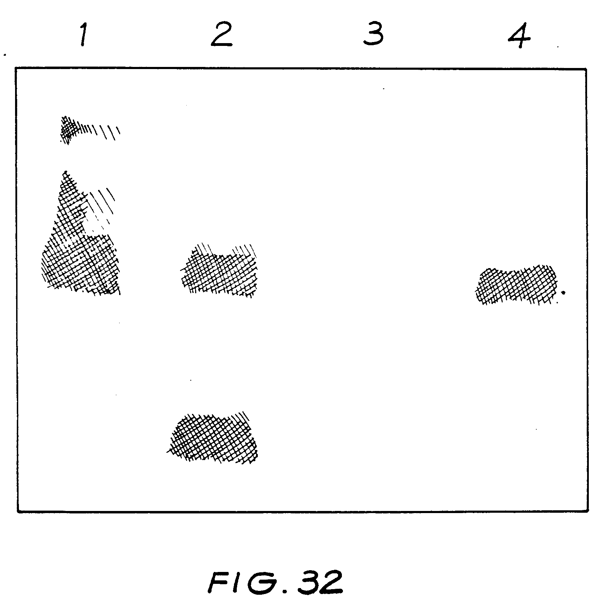

Colorimetric assays of culture supernatants from the same experiment as in Figure 32.

### GENERAL PROPERTIES OF MINACTIVIN

Minactivin is heat labile above a temperature of 56°C but is stable to freezing at -20°C and thawing (0-5% of its activity is lost after one cycle). Minactivin containing supernatants collected under sterile conditions can be stored at 4° in the presence of gentamicin for two months without significant loss of activity.

The interaction of minactivin with plasminogen activator does not appear to be ionic strength dependent, although ionic strength does have an inverse effect on plasminogen activation (19). While the activity or urokinase in the colorimetric assay was strongly inhibited by increasing salt concentration, the effect of minactivin on the diminished urokinase activity was proportionally the same.

Minactivin is relatively stable in the pH range 5 to 9 at 4°C. Propanol and methanol (greater than 10%) completely abolish minactivin activity.

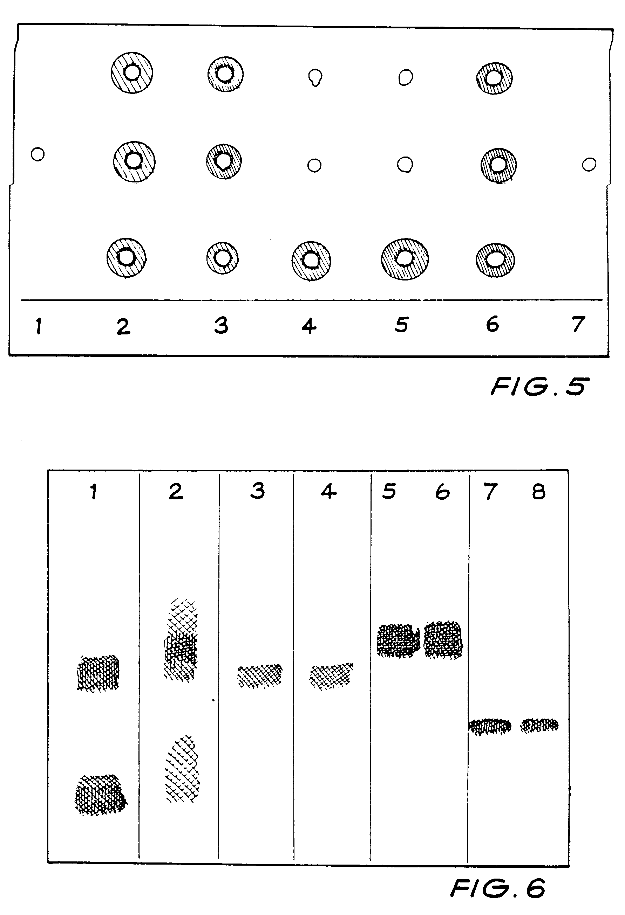

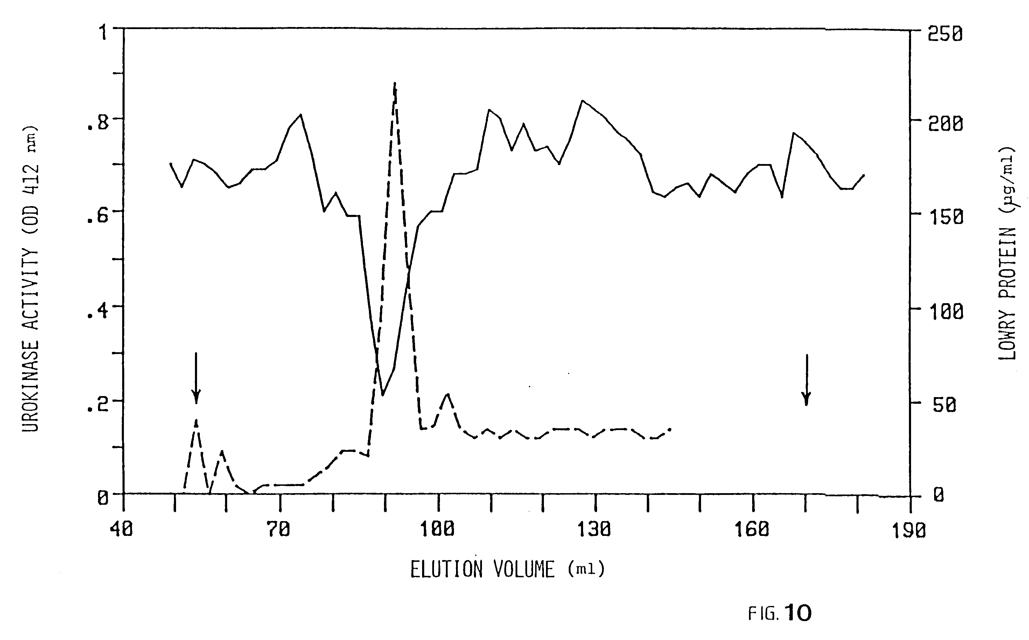

Gel filtration of minactivin preparations on Sephacryl S300 (Pharmacia) in the presence of salt show an apparent molecular size for minactivin identical to that of the serum albumin standard, i.e.. 60-70,000 (Fig. 10). Analysis of the specificity of the enriched minactivin preparation obtained from this column by SDS-PAGE-fibrin overlay (as described above), gave identical results to those obtained in Figure 6 for minactivin supernatants derived from monocyte culture. The observed inactivation of urokinase was not, however, a property of serum albumin since (a) human serum albumin had no effect on the colorimetric assay of urokinase, (b) the minactivin activity was not retained by an albumin affinity column of blue-Sepharose (Pharmacia), (c) the media concentration of human serum albumin would not have been expected to change with muramyl dipeptide or cell type in the induction experiments above and (d) cultures of monocytes without albumin or serum supplement also produced minactivin.

### ROLE OF MINACTIVIN IN TUMOUR DIAGNOSIS AND TREATMENT

It is well established that several major human carcinomas produce significantly enhanced levels of urokinase-type plasminogen activator (HPA52) in contrast to the levels found in normal tissues. Recent extensive studies of Markus and his coworkers have included tumours of lung, prostate, breast and colon (30-32), with attention to enzyme content and type, as well as the rate of secretion by tissue explants in short-term culture (34-35). It has further been established that HPA52 is a different gene product (36), not derived by proteolytic or other modification of the tissue-type plasminogen activator (HPA66) which is ubiquitous in all vascularized tissues (37), such as colon endothelial cells. It is clear that a change of gene expression occurs in epithelial cells during development of human colon cancer, leading to higher levels of HPA52.

Biological processes in which cellular plasminogen activators have been implicated involve those physiological events associated with invasion and tissue destruction, such as inflammatory reactions and tumour metastasis (38). Specific actions in which plasminogen activators are involved which relate to its role as a mediator of tumour invasion include its ability to stimulate cell division (39), modify cell surfaces (40), enhance cellular migration (41), digest fibrin surrounding tumours (42), and also activate collagenase (43).

As minactivin has been shown to be a potent specific inactivator of urokinase-type plasminogen activator (HPA52 and HPA33), it follows then that minactivin has a range of applications as a clinical reagent, diagnosis of chronic inflammation and various types of carcinomas, as well as in the treatment of these conditions. By specific modulation of HPA 52 activity in vivo, minactivin would have no effect on the role of tissue-type plasminogen activator (HPA66) in the maintenance of haemostasis. Furthermore, as minactivin is a natural product, it has the advantage of lack of antigenicity and should avoid clearance problems within the body.

Minactivin, therefore has application as an index for inflammatory disease status. Currently chronic inflammatory conditions are treated with steroids, but it is generally difficult to monitor the progress of the treatment. Using an antibody to minactivin, the activation state of the macrophages in body fluids and tissues may be monitored and hence the progress of treatment. Inflammation or chronic ulceration and damage to the mucosal surface cell layer, or epithelium, results in the infiltration of large numbers of blood monocytes into the damaged area. In this environment, the monocytes may be stimulated to produce minactivin in amounts related to the extent of inflammation.

Furthermore minactivin has application as a reagent for identifying and defining tumour boundaries both in histological specimens and in vivo. While solid tumours are generally re-dressed through surgery, the use of minactivin would enable the identification of small metastatic cancers arising following surgical intervention. In the analysis of histological specimens, minactivin, or its antibody, may be labelled with an isotope, such as I¹³¹, or conjugated to an appropriate enzyme or other chemical reagent. On contact with a histological specimen, minactivin will bind to the tumour-type plasminogen activator at its place of secretion, thereby identifying the tumour boundaries. Visualization of the complexed minactivin can then be effected by known procedures (44-47). For imaging tumours in vivo minactivin may be labelled with an appropriate isotope such as technetium⁹⁹, and after administration, the location and boundaries of the tumours determined by known radioisotopic methods (47-50). Moreover, as minactivin binds only to the active form of plasminogen activator, it would target tumour cells in growth or invasive stages.

In addition to its diagnostic applications, minactivin is also indicated for use in the direct treatment of tumours. As a specific inhibitor of the enzyme implicated in the process by which tumours invade surrounding tissues, regulation and, in particular, inhibition of tumour growth and metastases can be achieved. Furthermore, minactivin may be used as a drug delivery system to deliver lectins or toxins directly to growing tumours. It will be appreciated that this would offer many advantages in terms of specificity and extremely potent tumouricidal capability.

Further details of the production, purification and identification of minactivin are given in the following examples in association with the accompanying drawings and is not in any way intended to limit the scope of the invention.

### PREFERRED EMBODIMENTS of the INVENTION

### EXAMPLE 1

### BIOLOGICAL IDENTIFICATION AND CHARACTERIZATION OF MINACTIVIN

### Methods

### 1. Colorimetric Assay of Plasminogen Activators

The method of Coleman and Green (12) was used to assay several types of plasminogen activator enzymes in an assay buffer of 50mM glycine pH 7.8 containing 0.1% Triton X-100 and 0.1% gelatin. Dilutions were used which kept activity in the range 2-8 mPlough units (mPU) per assay. Enzyme (10ul), plasminogen (0.1 mg/ml, 20ul) and assay buffer (20ul) were incubated for 45 min at 37°, then plasmin assay reagents (1 ml, see ref. 12) were added and incubated for further 30 min at 37°. The reaction was stopped by the addition of Trasylol (20ul at 0.3mg/ml), and the absorbance monitored at 412nm. Human serum albumin (1%), used to supplement RPMI for monocyte culture, had no effect on the assay of urokinase by this method.

Human plasminogen was purified from pooled fresh plasma by two cycles of affinity chromatography on a lysine-Sepharose (Pharmacia) column (13), and was used in the assay at a final concentration of 0.3uM. Two commercially available preparations of human plasminogen activator were used: Urokinase, Calbiochem reference standard (lot 103285, 1700 Plough units/vial) from Calbiochem-Behring Corp. La Jolla, CA. and Sigma urokinase (lot 101F-05991, approx. 40,000 Plough units/mg protein). These are referred to in this paper as CUK and SUK respectively. Both preparations were shown by SDS-PAGE and fibrin overlay development (see below) to be a mixture of the plasminogen activator of Mᵣ 52,000 (HPA52) with its proteolytic product of Mᵣ 36,000 (HPA36). Tumours of the rat mammary adenocaricinoma line 13762 (a gift from Dr I Ramshaw, Dept. Experimental Pathology, JCSMR) were homogenised in 50mM glycine pH 7.8 containing 0.5% Triton X-100. Bovine trypsin (type lX), bovine thrombin (Grade 1) and porcine plasma were obtained from Sigma.

### 2. Colorimetric Assays of Minactivin

The quantity of minactivin in culture supernatants and cell lysates was determined by the inhibition produced in colorimetric assays of urokinase reference standard (12). The samples (20ul) were preincubated with 4 mPU urokinase (20ul) for 90 min at 23° before addition of plasminogen. Controls were included to verify that inhibition was at the level of plasminogen activation and not simply inhibition of plasmin, by direct addition of the minactivin source to the assay of preformed plasmin. One unit of minactivin was defined as that amount which inhibited 1 Plough unit of urokinase.

### 3. Radial Diffusion Assays

Fibrinogen (Sigma type X), human plasminogen and thrombin (Sigma grade 1) were cast in a 1.25% agarose (Sea Plaque, FMC Corp., Rockland, ME USA) gel matrix 1.2mm thick. After clotting at 37°C the gel was hardened at 4°C. and 3mm wells were cut for the application of urokinase/minactivin mixture (5ul). The gels were incubated in a humidified box for 20 hours at 37°C, and the lysis produced was enhanced by extensive washing in saline, followed by staining with amido black.

### 4. SDS-PAGE and Fibrin Overlay Development of Plasminogen Activator Activity

The samples applied to SDS electrophoresis gels consisted of either the plasminogen activator enzyme alone in assay buffer, or the enzyme preincubated for 90 mins at 23° with culture supernatant and/or protease inhibitor, plus SDS sample buffer to give a final volume of 100ul. The protease inhibitors employed were Trasylol, alpha₁-antitrypsin (Sigma), soybean trypsin inhibitor (Sigma), iodoacetamide, EDTA, SDS, tranexamic acid (Aldrich Chemicals, Milwaukee WI) and benzamidine. The gel slabs were of 11% polyacrylamide and were run in the vertical position, washed in Triton X-100 and developed by contact lysis of a fibrin/agarose gel according to the method of Granelli-Piperno & Reich (13). The development time for the fibrin lysis was usually 5-8 hr at 37°C. The molecular weight of enzyme lysis bands was estimated by comparison with those of the commercial enzymes, and with the position of stained standard proteins (Pharmacia LMW Kit) in the acrylamide gel.

### 6. Morphology and Cytochemistry

For phase contrast microscopy, adherent monocyte or macrophage monolayers were washed twice and fixed in 1.5% glutaraldehyde in 0.1M cacodylate buffer (pH 7.4) for at least 30 minutes at room temperature (9) prior to examination.

Cytocentrifuge preparations were prepared in a Shandon Southern centrifuge. Briefly, 0.2ml aliquots containing 50,000 cells in 10% foetal bovine serum were spun for 3 minutes at 500 rpm. The preparation was then dried rapidly in a hair dryer and the preparations stained with May-Grunwald Giemsa.

The presence of non-specific esterase was assayed using alpha-naphthyl acetate as substrate (10,11).

Phagocytic activity was assayed by incubating adherent monolayers or cell suspensions in RPMI-1640 containing 10% human AB serum and 1.1um latex beads (Sigma) at a concentration of 100 beads per cell for 60 minutes at 37°C. Non-ingested beads were removed by washing monolayers or cell suspensioris. 150 x g for 10 minutes at 20°, three times with RPMI-1640. Cells ingesting two or more latex beads were judged to be phagocytic.

### 6. Monocyte and Macrophage Cell Preparations

### a) Human Blood Monocytes

Human mononuclear leukocytes were isolated on Ficoll-Hypaque (Pharmacia fine Chemicals. Sydney. Australia) gradients from buffy coats removed by the Red Cross Blood Transfusion Service of the Woden Valley Hospital. After four washes in Pi/NaCl at 4°C to remove contaminating platelets the cell pellet was resuspended in RPMI-1640 (Gibco, Grand Island, N.Y) containing 60 units/ml gentamicin. Purified monocyte monolayers were obtained by plating 10⁷ mononuclear cells in RPMI-1640 with 10% AB serum into 30mm wells (Linbro multiwell plates. Cat. No. 7605805) which had been precoated for 30 min with 2ml of human AB serum. Monocytes were allowed to adhere for 60 min at 37°C in an atmosphere 5% CO₂ in air. Non-adherent cells were then removed by washing monolayers six times with RPMI-1640 prewarmed at 37°C.

Adherent monolayers were shown to be greater than 85% monocytes by several criteria. Polychromatic staining of the monolayer cells showed 94 ± 6 percent of the cells to be monocytes having a large round or indented nucleus with basophilic cytoplasm. Staining for the cytoplasmic enzyme non-specific esterase showed monolayers to be 85 ± 2% positive for this monocyte marker. Ninety one percent of the adherent cells phagocytosed latex beads confirming that the adherent cell population which showed the morphological and histochemical appearances of mononuclear phagocytes also exhibited this characteristic functional property.

During in vitro culture, monocytes underwent pronounced changes in cell size and morphology. Cyto-centrifuge preparations of cultured monocytes showed that monocytes continued to increase in size throughout the culture period and by the end of seven days, many cells had more than doubled in apparent size. Occasional binucleated cells were also seen. As monocytes increased in size their cytoplasm became heavily vacuolated, and cytoplasmic granules also became evident. Further culture resulted in increased formation of multinucleated giant cells.

### b) Peritoneal Macrophages

Two litre lots of dialysate fluid, from patients undergoing routine peritoneal dialysis for chronic renal failure, were centrifuged at 300 x g for 10 minutes at 20°C. The cell pellet was resuspended in 50 ml of RPMI-1640 and recentrifuged for 10 minutes at 200 x g at 20°C The cells were then resuspended in RPMI-1640 to a final concentration of approximately 2 x 10⁶ per ml and 6 ml aliquots of the peritoneal cell suspension were underlayed with 3 ml of Ficoll-Hypaque and centrifuged for 20 minutes at 400 x g at 20°C. The cells were resuspended to a concentration of 1 x 10⁶/ml in RPMI-1640 containing 10% human AB serum.

High yields of mononuclear cells were obtained from Ficoll-Hypaque gradients of peritoneal washings. Forty-seven percent of the cells were macrophages as judged by morphologic criteria. Cytocentrifuge preparations of the mononuclear cell fraction showed that the macrophage population was heterogeneous and included large mature cells with abundant cytoplasm and occasionally two nuclei, as well as smaller cells more characteristic of monocytes, with indented nuclei and a higher nucleus to cytoplasm ratio.

Peritoneal macrophages were further purified by adherence to plastic culture trays as described above for monocytes. Following removal of the non-adherent cell layer, viable peritoneal macrophages could be maintained in culture for up to three weeks provided that the culture media was changed every 2-3 days. Peritoneal monolayers were routinely greater than 87% macrophages as determined by non-specific esterase staining.

### c) Bone Marrow-derived Macrophages

Fresh pieces of human ileac crest and rib marrow were obtained during orthopaedic procedures and dispersed in RPMI-1640 media containing 10% giant cell tumour (GCT) culture supernatant (Gibo-Biocult, Grand Island, NY, USA) and 10% foetal bovine serum. This primary culture was maintained for 7 days on gelatin-coated flasks (1). After this period, monocyte/macrophages were purified by adherence to culture dishes prepared as for blood monocytes and the secondary adherent culture used for minactivin measurements. The cells obtained were greater than 90% positive for non-specific esterase.

### d) Colonic Mucosa Macrophages

These cells were obtained from resected colons by enzymatic disaggregation of the colonic mucosa according to the method of Golder and Doe (2). They were grown as adherent monolayers in RPMI-1640 as for monocytes. The cells obtained were greater than 85% non-specific esterase positive and phagocytosed sheep red blood cells.

### 7. Cell Culture

### a) Monocyte and Macrophage

Monocyte or macrophage monolayers were cultured in RPMI-1640 with 1% human serum albumin (Commonwealth Serum Labs., Melbourne, Australia). Cultures of cells from sources (a)-(d) above were kept as close as possible to a ratio of 3 x 10⁶ cells/ml media.

The following agents were used in cell culture experiments:
(a) Muramyl dipeptide (N-acetyl-muramyl-L-alanyl-D-isoglutamine, Peninsula Laboratories, San Carlos, CA, USA) at 5ug/ml, (b) Salmonella minnesota R595 cell wall lipopolysaccharide (3). The latter was prepared by sonication of the water-insoluble extract in 0.1% triethylamine followed by extensive dialysis against Pi/NaCl. The final concentration in cultures was 0.1ug/ml. Dexamethasone and phorbol myrastate acetate (Sigma Chemical Co., St. Louis, MO. USA) were used at a final concentration of 0.1uM, and 10ng/ml, respectively.

### b) Transformed Cell Lines

The human macrophage-like cell lines U937 (4) and HL60 (5) were obtained from Drs R Ulevitch and R G Painter (Scripps Clinic, and Research Foundation, La Jolla, CA, USA). The U937 cell line was also obtained from the American Type Culture Collection (Rockville, Maryland, USA). RC2A (6) was from Dr N Kraft (Prince Henry's Hospital, Melbourne, Australia). MLA144 is a Gibbon ape T-lymphocyte line (7). The human erythroid line K562 (8) was a gift of Dr H S Warren, Cancer Research Unit, Woden Valley Hospital, while COLO 394 was a human colon carcinoma cell line provided by Dr R Whitehead, Ludwig Institute of Cancer Research, Royal Melbourne Hospital, Melbourne. All of the above cells were grown in RPMI-1640 medium without serum prior to harvest of the supernatants for analysis. Cell lysates were prepared in the presence of 0.5% Triton X100 for enzyme studies.

### BIOLOGICAL CHARACTERISTICS OF MINACTIVIN

### Results

### Minactivin Production in Cell Populations

### a) Human Blood Monocytes

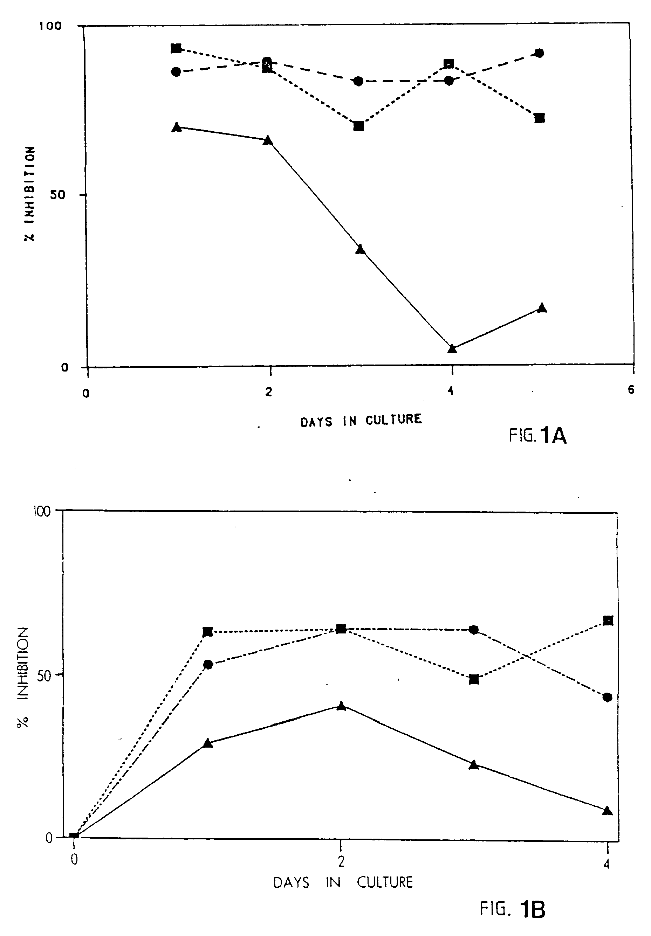

Minactivin was shown to be present in serum-free conditioned media from adherent human monocytes by inhibition of the activity of urokinase in the colorimetric assay of Coleman and Green (12) (Fig. 1). This inhibition was observed whether or not the initial adherence of the cells was facilitated by human serum. The non-adherent mononuclear fraction obtained during preparation of adherent monolayers did not produce any detectable minactivin in culture.

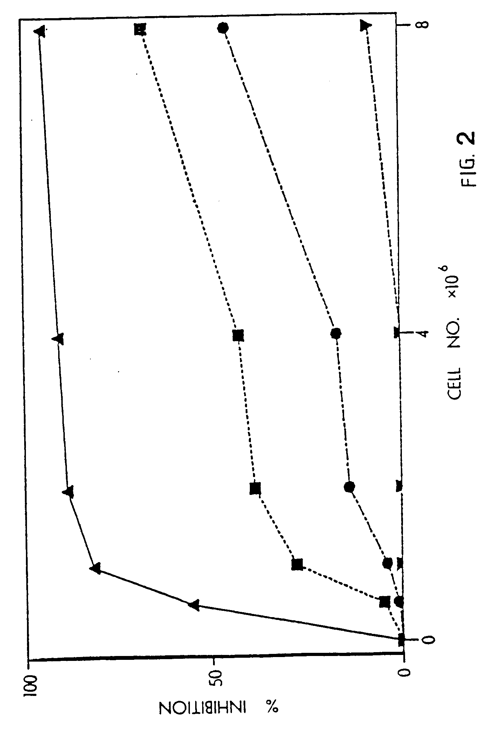

Maximal minactivin activity was obtained using culture supernatant from the first 24 hr of monocyte culture. Subsequently the rate of inhibitor production for each successive 24 hr period declined until it was insignificant after four days. This effect was observed in both the culture supernatants and cell lysates. However, activation of monocytes in vitro by muramyl dipeptide or bacterial lipopolysaccharide led to both sustained production and secretion of minactivin (Fig. 1A and 1B). The amount of inhibitor in the culture supernatant from the first 24 hr period was correlated with cell number (Fig. 2). Maximum levels of minactivin were produced in culture by cells in densities of greater than 10⁶ cells/ml. Relatively lower amounts of minactivin were produced (per cell) at higher cell densities. Treatment of monocyte cultures with dexamethasone (10⁻⁶ - 10⁻⁸ M) which has previously been shown to induce synthesis of an inhibitor of plasminogen activator in human fibroblast cultures (14), had no effect on the production of minactivin.

### b) Human Peritoneal Macrophages

While lysates of freshly isolated blood monocytes did not contain minactivin (see Fig. 1B), all lysates of freshly isolated adherent peritoneal macrophages contained significant levels of minactivin (approx. 30% inhibition), which subsequently increased during culture (Fig. 3). This sustained production of minactivin was also reflected in the high levels of secreted product measured in the corresponding culture supernatants. Unlike control monocyte cultures, peritoneal macrophage lysate minactivin levels remained high throughout the 3 day culture period. This cell population appeared from its morphology to consist largely of newly recuited monocytes, indicating that the in vivo stimulus of mild inflammation induced by repeated dialysis was sufficient to recruit and activate monocytes to produce minactivin within the peritoneal cavity. The peritoneal macrophages appeared to be unresponsive to further activation, since addition of muramyl dipeptide did not further enhance minactivin production in either the lysates or the supernatants (Fig. 3)

### c) Bone Marrow-Derived Macrophages

Bone marrow macrophages were obtained as the adherent cells after 7 days of GCT (giant cell tumour cultures supernatant) stimulated growth of primary marrow cultures. Since these cells could only be studied as secondary cultures after the 7 day primary culture of the tissue, their initial minactivin production and secretion can not be directly compared to freshly isolated blood monocytes or peritoneal macrophages.

Nonetheless, after primary culture for seven days, the lysates of adherent bone marrow macrophages obtained during secondary culture showed high levels of minactivin activity which declined regardless of the presence or absence of muramyl dipeptide (Fig. 4). These bone marrow cells were also found to secrete high levels of minactivin into supernatants, and as observed with the lysates, this level was not affected by muramyl dipeptide (Fig. 4).

### d) Colonic Mucosal Macrophages

Macrophages isolated from enzymically disaggregated mucosa of resected human colon had only very limited ability to produce minactivin in culture, and the levels were insignificant compared to those from the three sources above (mean value of 4% inhibition for 24 hr supernatants in four separate experiments). These low levels were not increased when Salmonella minnesota lipopolysaccharide (0.1ug/ml) was added to cultures.

To determine if this lack of responsiveness of intestinal macrophages could be attributed to the prolonged exposure to the degradative enzymes (collagenase and DNAase) used in their isolation, some normal human blood monocytes were subjected to several hours incubation in the supernatant medium from an intestinal macrophage preparation. The enzyme treated blood monocytes retained their ability to produce and secrete minactivin in subsequent culture (see Table 1).

**TABLE 1**

| | Control Monocytes | Enzyme Treated |
|---|---|---|
| Lysate | 90% | 100% |
| Supernatant | 67% | 72% |
| Production of minactivin by human blood monocytes before and after incubation with colon disaggregation supernatant containing DNAse and collagenase. The results are expressed as per cent inhibition of urokinase assay by monocyte supernatant. | | |

Thus, it appears that blood monocytes are at an ideal developmental stage for potential secretion of minactivin. This potential is realised when they are activated with muramyl dipeptide in vitro, or actively recruited to an inflammatory site in vivo. On the other hand, mature tissue macrophages isolated from intestinal mucosa appear to have lost their ability to produce and to secrete minactivin, even upon stimulation.

### e) Human Macrophage Cell Line U937

Minactivin activity could not be detected in supernatants obtained from the human macrophage cell line U937 when assayed by the method of Coleman and Green (12). However, when this cell line was cultured in the presence of dexamethasone (1uM), significant levels of minactivin were measured in the culture supernatants. This result may be explained by the concomitant production of plasminogen activator by U937 cells which would effectively bind the minactivin produced, thereby masking its detection by colorimetric assay. Indeed, when the U937 cells were removed from dexamethasone containing media for 72 hours, high levels of intracellular plasminogen activator were induced as measured by assay of plasminogen activator activity in 0.5% Triton X100 cell extracts. U937 cells cultured for greater than 7 days in the absence of dexamethasone completely lost their ability to produce minactivin. Addition of dexamethasone, even to 10uM final concentration, did not restore minactivin production.

The level of minactivin secreted constitutively by U937 cells was approximately 4-fold lower than that produced by muramyl-dipeptide induced monocytes in culture. However, the level of Minactivin Secreted by U937 cells could be enhanced 16 fold by the addition of phorbol esters, such as phorbol myristate acetate, to the cells in culture. Minactivin activity was not detected in supernatants obtained from the human macrophage-like cell line RC2A or the T-lymphocyte cell lines of human (Jurkat) and Gibbon ape (MLA144) origin.

### 2. Minactivin Specificity

### a) Inhibition of Fibrinolysis

The specificity of minactivin inhibition of proteases was investigated by incubating minactivin containing monocyte culture supernatants with various enzymes added to microwells cut in a fibrin/agarose gel. Plasminogen (20ug/ml) was included in the gel mixture to enable plasminogen activator expression and suitable concentrations of enzyme were chosen to give comparable areas of lysis after incubation for 20 hours at 37° (see description of Fig 5).

Minactivin was clearly not an inhibitor of plasmin or trypsin (Fig 5), even when preincubated with these enzymes for 2 hrs at 23° before application to the wells. Among human plasminogen activators, both preparations of urokinase (CUK and SUK) lost all fibrinolytic activity when treated with minactivin. However, the fibrinolytic activity of human melanoma culture supernatant, due almost entirely to tissue-type plasminogen activator (HPA66) (see below), was not inhibited by minactivin, even after preincubation. The clotting of fibrinogen by thrombin was also unaffected by minactivin.

### b) Inhibition by Colorimetric Assay

The Coleman and Green (12) assay was further applied to investigate the specificity of minactivin inhibition. In the absence of plasminogen, incubation with plasmin assay reagents (12) can be used as a highly sensitive assay for trypsin as well as plasmin, while in the presence of plasminogen several types of plasminogen activators can be assayed. A notable exception however is HPA66 in human melanoma conditioned media, which was inactive in this assay, presumably due to the lack of fibrin (16).

Minactivin did not inhibit the lysine thioesterase activity of trypsin or plasmin (Table 2). However, the plasminogen dependent activity of the urokinase-type plasminogen activators, CUK and SUK and APA52 from a Gibbon ape T-lymphocyte line (MLA 144), were strongly inhibited by minactivin. Furthermore, the inhibition of urokinase was observed only when minactivin was present during incubation with plasminogen, and did not occur if minactivin was added at the plasmin assay stage.

The activity of rat plasminogen activator (RPA48, probably homologous to HPA52) present in the diluted homogenate of rat 13762 tumour was not affected by minactivin.

| Enzyme or Source of PA | Amount | Control Activity (A₄₁₂) | Minactivin treated (A₄₁₂) | % of Control Activity |
|---|---|---|---|---|
| Trypsin | 6ng | 0.32 | 0.31 | 97 |
| Plasmin | 180ng | 0.94 | 0.98 | 104 |
| CUK | 4mPU | 0.72 | 0.00 | 0 |
| SUK | 8mPU | 0.88 | 0.00 | 0 |
| Melanoma | 20ul supnt | 0.05 | 0.07 | - |
| MLA144 | 20ul supnt | 0.69 | 0.08 | 12 |
| Rat tumor-13762 | 20ul 1%-homog. | 0.53 | 0.50 | 94 |
| Inhibition of Protease colorimetric assays by minactivin. Undiluted minactivin culture supernatant (20 ul) was preincubated for 2 hrs at 23° with the enzymes indicated in a final volume of 40 ul. Plasminogen (20 ul, 100 ug/ml) was then added to the activators, plasmin assay reagents (1 ml) were added to the other proteases, and the respective assays continued to colour development. | | | | |

### c) SDS-PAGE and Fibrin Overlay

The fibrin overlay method of Granelli-Piperno & Reich (14) was used to verify the specificity of minactivin inhibition and to determine whether the various plasminogen activators were irreversibly inactivated by minactivin. Preincubations of enzymes with minactivin were subjected to SDS-PAGE, and residual enzyme activities were visulaized as lysed areas on a fibrin/agarose gel supplemented with plasminogen.

When the acrylamide and fibrin gels were incubated together for 5 hrs, the control (untreated) urokinase (CUK) gave two prominent bands: one characteristic of HPA52 and a second characteristic of its proteolytic product (17), HPA36 (Fig 9. lane 1). However if CUK was preincubated with minactivin before SDS-PAGE, a single weak lysis band appeared in the overlay gel after 5 hrs, and this was located at a higher molecular weight position that HPA52 (Fig 9A, lane 10).

When the gels were incubated together for 20 hrs, this band increased in intensity, and an additional band became visible at a higher apparent molecular weight than that observed for HPA36 (see Fig 6, lane 2). These results demonstrate that minactivin inactivates HPA52 and HPA36 in such a way that the final residual activities are of higher molecular weight than the untreated enzymes. Thus, it appears that the mode of interaction between minactivin and urokinase may involve complex formation or a radical change in the structure of urokinase affecting its electrophoretic mobility (see Section 3 below). HPA36 appeared to be more susceptible to inactivation than HPA52.

The Gibbon ape cell line MLA 144 secreted plasminogen activator into serum free culture media, and after SDS-PAGE and fibrin development, this activity was resolved into two closely spaced bands in the same molecular weight region as the HPA52 from human urokinase (Fig 6, lane 3). Neither of these bands was affected by preincubation of the MLA 144 culture supernatant with minactivin (Fig 6, lane 4). The plasminogen activator of Mᵣ 66,000 from human melanoma culture supernatant (Fig 6, lanes 5 & 6) and that of Mᵣ 48,000 from a rat tumour were also unaffected (Fig 6, lanes 7 & 8) by treatment with minactivin. These results demonstrate that minactivin specifically inhibits human urokinase-type plasminogen activators (HPA52 and HPA36), and not human tissue-type plasminogen activator (HPA66), nor plasminogen activators from (at least some) other mammalian species.

### 3. Interaction of Minactivin with Urokinase-type Plasminogen Activator

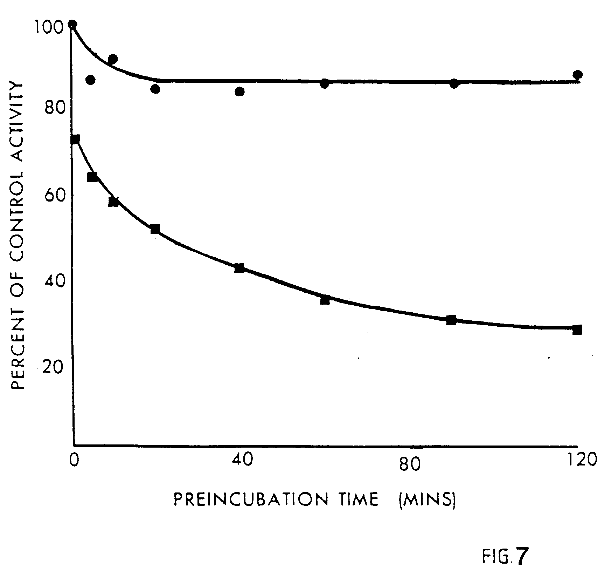

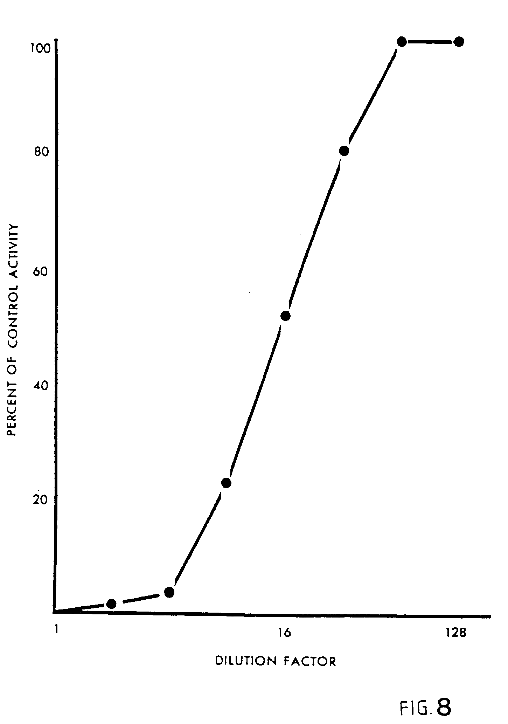

The interaction between minactivin and urokinase was analysed by preincubation of minactivin and CUK in the absence of plasminogen, followed by assay of the residual plasminogen activator activity in the colorimetric assay described above. Inactivation took place over a time course of two hours at 23°, and the final level of residual plasminogen activation activity was less than half that observed when urokinase, minactivin and plasminogen were mixed at zero time (Fig 7). During the two hour preincubation, inactivation was initially rapid and then plateaued, so that further incubation resulted in no inactivation of the residual urokinase. This result suggested that the reaction was stoichiometric, rather than that, for example, the urokinase was degraded by a protease. Titration of urokinase with dilutions of minactivin containing culture supernatant produced a simple interaction curve shown in Fig 8. The activity of 4mPU of urokinase (CUK) could be 50% inactivated by the equivalent of 1.5ul of the culture supernatant used for this experiment.

The culture supernatants used for these experiments was devoid of any detectable general proteolytic activity, as shown by the control well in the fibrinolysis experiment above (Fig 5), and by the lack of detectable lysine thioesterase activity in the highly sensitive colormetric assay. However, to determine whether the inactivation of urokinase by minactivin involved proteolytic activity on the part of either of these two molecules, CUK was preincubated with and without minactivin, and in the presence of each of a wide range of protease inhibitiors. The protease inhibitors and their final concentrations were: Trasylol. 0.2mg/ml; alpha₁-antitrypsin, 16ug/ml, soybean trypsin inhibitor 0.16mg/ml; iodoacetamide. 3mM; EDTA, 6mM; SDS, 0.6%; tranexamic acid, 3mM and benzamidine, 3mM. These preincubations were then subjected to SDS-PAGE and the urokinase activity visulaized by fibrin lysis as above. Control preincubations with each of the protease inhibitors in the absence of minactivin showed that only soybean trypsin inhibitor abolished the usual HPA52 and HPA36 bands visible after electophoresis (Fig 9A). When minactivin was included in each preincubation, none of the other protease inhibitors prevented the inactivation of urokinase catalysed by minactivin (Fig 9B). However, SDS was able to prevent inactivation when added to the urokinase before or immediately after minactivin (Fig 9, lane 8). Thus minactivin is demonstrated to be an inactivator of plasminogen activator, rather than an inhibitor.

Taken together, these results suggest that the mode of action between minactivin and urokinase involves the initial rapid formation of a reversible complex, followed by a slower SDS-sensitive phase (insensitive to protease inhibitors) which yields an irreversible product of higher molecular weight than the free enzyme but lower than that expected for a 1:1 complex of enzyme and minactivin. Thus, SDS added to the preincubation at zero time was able to completely reverse the inhibitory effect of minactivin, whereas preincubation with minactivin followed by treatment with SDS resulted in complete inhibition of both HPA52 and HPA36. Trace activities appeared at positions of higher molecular weight after prolonged development, which could be inhibited if minactivin was added to the fibrin overlay. These may represent small amounts of partially activated enzyme or a dissociation product from the initial reversible complex formation.

A number of plasminogen activators from transformed cells of the human macrophage lineage were also tested for minactivin inactivation by colorimetric assay. It was clear that minactivin strongly inhibited the plasminogen activators from the human macrophage precursor cell line (HL60) and the human macrophage-like leukemic cell line (RC2A) (Table 3). The plasminogen activators secreted by the human erythroid cell line (K562) and the primate T-lymphocyte cell line (MLA 144), were also inhibited, but not the urokinase of mouse urine.

| PA Source | % Inhibition of 2mPU |
|---|---|
| Urokinase equivalent | |
| Urokinase | 95 |
| Monocyte | 18 |
| RC2A | 80 |
| HL60 | 78 |
| K562 | 75 |
| MLA144 | 60 |
| Mouse urine | 4 |
| Inhibition by monocyte minactivin of plasminogen activators from various sources, including human transformed cell lines. | |

### 4. Specificity of Minactivin Obtained from Other Cell Populations

Confirmation that the minactivin produced by other macrophage cell populations was identical to minactivin produced by monocytes was obtained using fibrin radial diffusion assays, as monocyte minactivin is highly specific for human urokinase type plasminogen activators.

A selection of protease and plasminogen activators, including plasmin, trypsin, HPA66 (tissue-type activator from human melanoma culture supernatant) and human urokinase (both HPA52 and HPA36) were tested for inhibition by minactivin produced in each cell population.

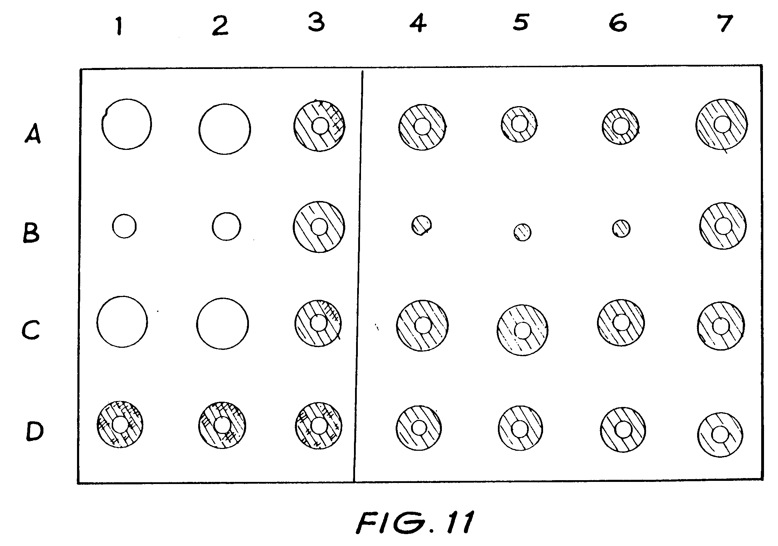

Specific inhibition of urokinase-type plasminogen activator was observed using culture supernatants from both peritoneal macrophages and blood monocytes (Fig 11). The minactivin produced by these cells had no effect on the activities of plasmin, trypsin or HPA66. Total cell lysates of peritoneal macrophages, blood monocytes and bone marrow macrophages which had been solubilized with Triton X100, all produced inhibition of urokinase-type plasminogen activator, with no detectable inhibition of plasmin or trypsin. The lysates of blood monocytes and peritoneal macrophages did, however, show some weak inhibition of HPA66 (see Fig 11).

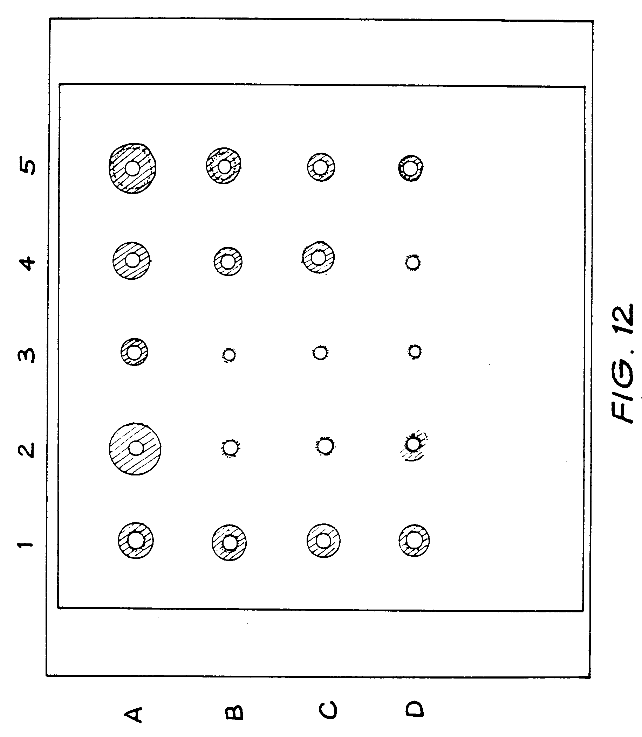

The minactivin produced by U937 cells was found to be identical to monocyte derived minactivin in its general characteristics. A comparison of their specificities by fibrin radial diffusion using purified minactivin from both U937 cells and induced monocytes demonstrated that minactivin from both sources specifically inhibited the urokinase-type plasminogen activators, CUK and SUK, and not tissue-type plasminogen acvtivator (HPA66) (Fig 12). No inhibition was observed in the plasmin and mouse urokinase controls.

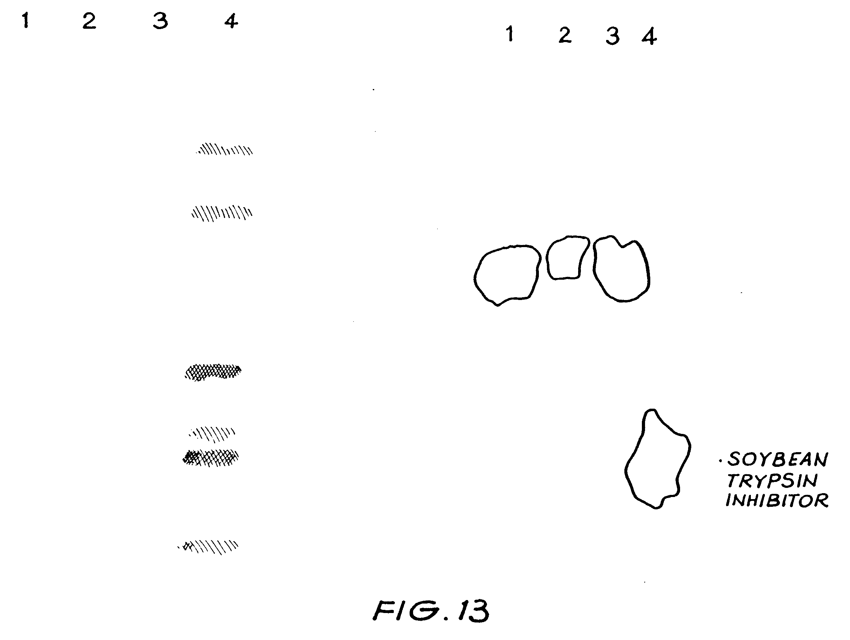

The electrophoretic mobilities of the monocyte and U937 cell derived minactivin preparations were very similar, when compared on a non-denaturing, discontinuous polyacrylamlde gel system (see legend Fig 13). The minactivin inhibition bands were visualized by incubation of the gel with urokinase, followed by fibrin/agarose gel overlay. The minactivin preparation from the U937 cell line appeared to have a slightly greater electrophoretic mobility than that derived from monocytes (figure 13). This minor difference may reflect a difference in the amount of lysis detected on the gel or may be due to a difference in glycosylation of the minactivin from the two different sources.

Minactivin isolated from U937 cells or induced monocytes differs in specificity from the plasminogen activator inhibitor isolated from human placenta (23) as demonstrated by fibrin radial diffusion (Fig 12). The human placental inhibitor (Calbiochem) inactivates both urokinase-type and tissue-type plasminogen activators, whereas minactivin inhibition appears to be specific for the former.

### EXAMPLE 2

### PROCESS FOR THE PURIFICATION OF MINACTIVIN

### 1. Purification of Minactivin from Human Monocyte Cultures

Monocytes were isolated form human blood by first centrifuging to form a white "buffy coat" of the leukocytes on top of the erythrocytes. The leukocytes were separated into granulocytes and lymphocytes plus monocytes by layering on Ficoll-Hypaque. The monocytes were separated from the lymphocytes by centrifugal elutriation.

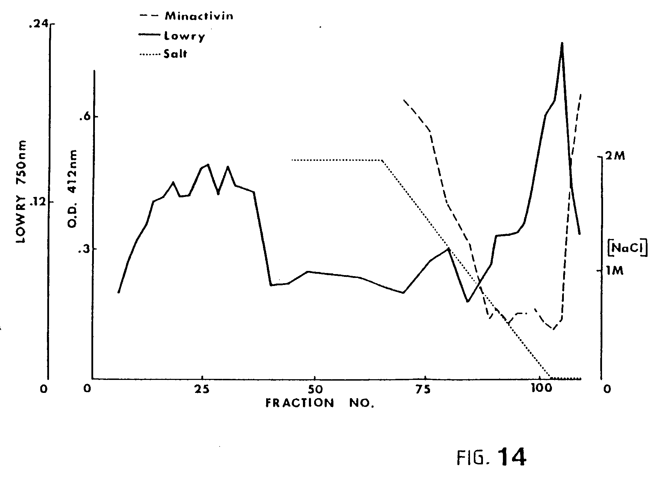

The purified monocytes (typically 5-8 x 10⁸ cells from 3-4 litres of blood) were adhered to 15cm plastic dishes (pretreated with gelatin, then human serum and washed) at a density of 0.20 - 0.34 x 10⁶ cells/cm² and cultured with serum-free RPMI-1640 (0.50 - 0.75 ml/10⁶ cells) containing 0.05 ug/ml of muramyl dipeptide (adjuvant peptide). These cultures were continued for 3 days, after which the media was harvested and clarified by centrifugation. Solid sodium chloride was then added to the supernatant to 2M, and the solution applied to a phenyl-Sepharose affinity (Pharmacia) column at 4°C. The column was washed with two bed volumes of 50 mM glycine, pH 7.8 containing 2 M NaCl to remove unbound proteins and then the bound proteins eluted with a descending gradient of NaCl in the same buffer. Fractions from this gradient were assayed for minactivin activity by the method of Coleman and Green (12) and the protein concentrations were determined by Lowry (24). The typical profile for this column is shown in Fig. 14. The most active fractions were pooled dialysed against 50 mM glycine pH 7.8, and applied (either without further treatment, or after concentration with a Centricon 30 (Amicon)) to an affinity column of Cibacron Blue-Sepharose (Pharmacia) which was equilibrated in the same buffer. Minactivin was not absorbed whereas other protein contaminants were retained by this column. The overall purification achieved by this procedure was of the order of. 1000 fold, based on protein concentration (Lowry method) and titration of minactivin activity by colorimetric assay of urokinase inhibition. The interaction between the minactivin product and urokinase was stoichiometric, as previously observed for the minactivin culture supernatants (see Fig. 8). Although typically the minactivin product contained 200-500 units of minactivin per ml, the minactivin component could not be unequivocally identified after SDS-polyacrylamide gel electrophoresis.

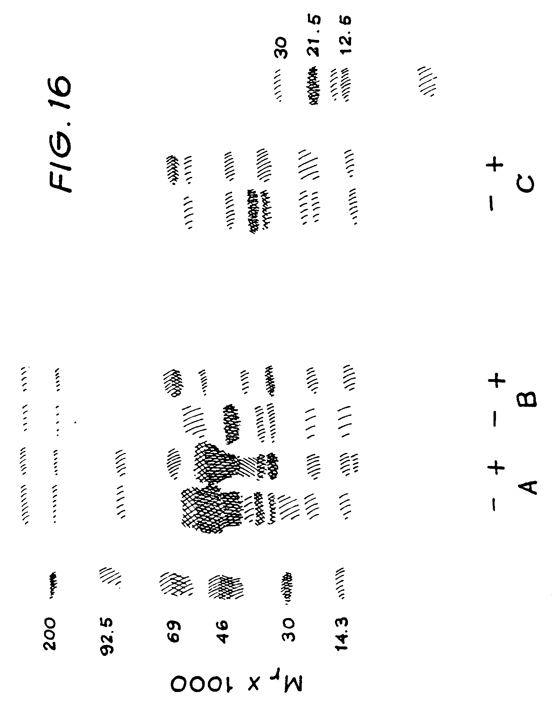

The purification of minactivin by this method was followed by SDS-polyacrylamide gel electrophoresis using in vivo labelled (³⁵S-methionine) monocyte preparations in culture. A number of proteins were secreted into the serum-free culture medium over the three day time course and a significant increase in specific activity was obtained following Phenyl-Sepharose chromatography (Fig. 15). Additional protein contaminants are further removed following chromatography on Blue Sepharose (Fig. 16). When urokinase (HPA36) was incubated with these minactivin preparations, a shift in molecular weight was observed yielding a urokinase-minactivin complex corresponding to a Mᵣ of 70-75,000 (Fig. 16). This change in electrophoretic mobility can be used to identify the molecular weight of the protein that is minactivin. The shift correlated with the disappearance of a major protein band in the minactivin preparation of apparent Mᵣ 39-48,000. Minor bands were also observed at Mᵣ 60,000 and 32,000. Under reducing conditions, a single protein band was apparent corresponding to a molecular weight of 30-35,000.

### 2. Purification of Minactivin from the Human Macrophage Cell Line, U937

### Cell Culture

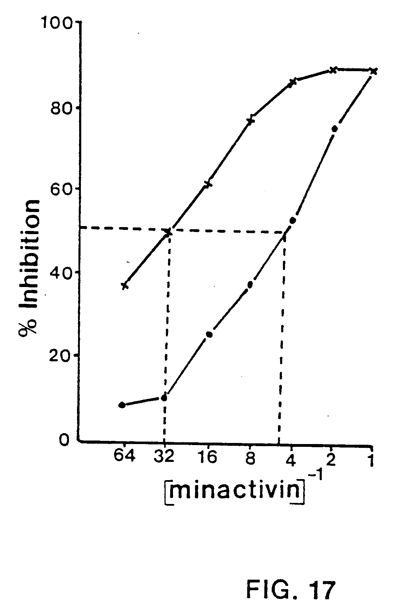

The nonadherent human macrophage cell line, U937, was cultured in RPMI 1640 containing 10% foetal calf serum and 1 uM dexamethasone, either in T175 culture flasks or in a 10 litre Braun fermenter. The cells were maintained at densities of 1-3 x 10⁶ cells/ml. Although minactivin was secreted by the cells during this growth phase, the cells were transferred to serum-free medium to obtain supernatants for minactivin purification. The cells were pelleted, washed once, resuspended in RPMI 1640, containing 1 uM dexamethasone, and cultured for a period of three days. For some of the purification examples given below, 0.7% gelatin was added to the serum-free medium to enhance cell viability. A 4-8 fold increase in minactivin activity was typically obtained in the presence of gelatin (Fig. 17).

The cells were then harvested and the supernatants used in the purification examples which follow.

### Purification Example 1: Phenyl-Sepharose Chromatography using a Step pH Elution

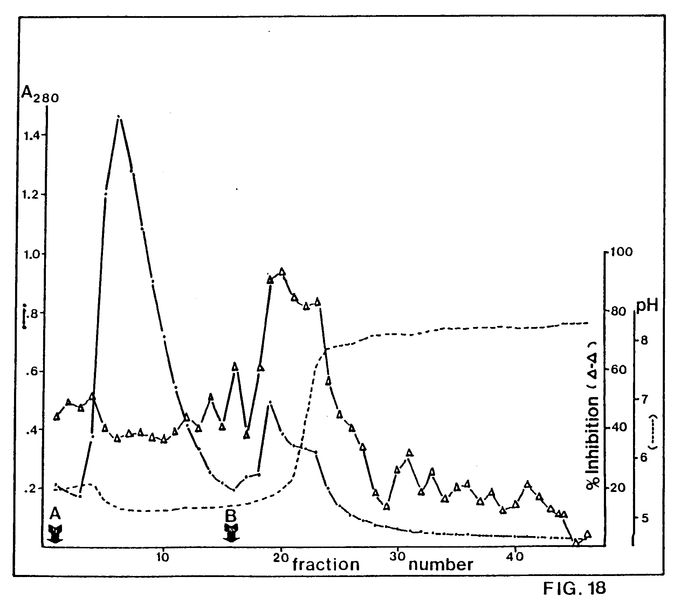

Minactivin was purified from supernatants obtained from U937 cells cultured in the presence of 0.7% gelatin. A 50 ml column of Phenyl-Sepharose was equilibrated with 50 mM citrate, pH 5.5, containing 2 M NaCl. 1.6 litres of U937 culture supernatant was adjusted to pH 5.5 with 1 M citric acid and solid NaCl added to a concentration of 2 M prior to application on the column. The column was then washed thoroughly with the equilibration buffer, and then with 50 mM citrate, pH 5.0, containing 0.5 M NaCl. Minactivin was then eluted with 50mM glycine, pH 7.8, and assayed for activity by the colorimetric method of Coleman and Green (12). Protein content was determined by the Bradford method (25). A typical column profile is shown in Fig. 18.

The yield was 3,360 units of minactivin or 66% having a specific activity of 140 units/mg. This represents a 214 fold increase in specific activity.

### Purification Example 2: Batchwise Phenyl Sepharose Chromatography using Salt Elution

Minactivin was purified from supernatants obtained from U937 cells cultured in the absence of gelatin unless otherwise specified.

### a) Concentration of Serum Free Minactivin Supernatants.

Typically, 4-5 litres of culture supernatant was concentrated 10-fold using an Amicon DC2 Hollow Fiber Dialysis/Concentration unit equipped with a 30,000 MW cut-off cartridge. The concentrate was then dialysed against at least an equal volume of 50 mM glycine, pH 7.8, to remove all traces of dye.

### b) Centrifugation of Minactivin Concentrate

The dialysed concentrate was centrifuged in a JA10 rotor at 8,000 rpm for 30 min at 4°C to pellet residual cell debris and protein that may have precipitated during dialysis. The clarified supernatant is then aliquoted and frozen at -20°C until required for subsequent purification.

100% of the initial minactivin activity is recovered at this stage, yielding typically 10-20,000 units of minactivin with a specific activity of 20 units/mg.

### c) Batchwise Phenyl-Sepharose Chromatography

Minactivin was further purified from the concentrated culture supernatant (obtained from cells cultured in the presence of 0.7% gelatin, specific activity, 1 unit/mg) by batchwise salt elution using Phenyl-Sepharose. The ionic strength of the supernatant (750 mls) was adjusted to 2 M with solid NaCl. Phenyl Sepharose which had been pre-equilibrated in 50 mM glycine, pH 7.8, 2 M NaCl, was added to the supernatant in a ratio of 1:7.5 (w/v). The suspension was allowed to incubate with agitation for 2 hours at room temperature or overnight at 4°C. The phenyl-Sepharose was removed by filtration under vacuum using a glass-scintered filter, and then washed thoroughly using the same apparatus with the equilibration buffer, followed by buffer containing 50 mM glycine, pH 7.8, 1.4 M NaCl to remove contaminating proteins. The minactivin was then eluted from the column with 50 mM glycine, pH 7.8

The yield of minactivin activity by this method was 4,800 units which corresponds to a 35% yield from the starting material. The specific activity of the minactivin product was increased by approximately 100 fold to 96 units/mg.

### Purification Example 3: DEAE-Sepharose Chromatography using Step-wise pH Elution

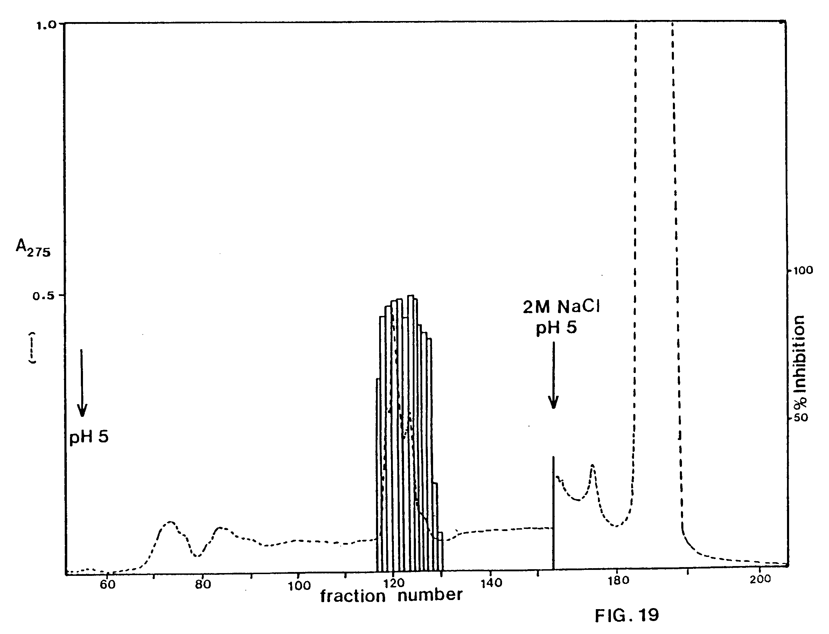

Cell free supernatants were processed through steps (a) and (b) as described in Purification Example 2 using pH elution. The concentrated minactivin supernatant (40 ml, 6.5 units/mg), was applied to a DEAE-Sepharose column which had been pre-equilibrated with 50 mM glycine, pH 7.8. After thorough washing with the equilibration buffer to remove unbound protein, minactivin was eluted using 25 mM citrate-phosphate, pH 5.0. This was followed by a wash with 25 mM citrate phosphate, pH 5.0 containing 2 M NaCl to remove any residual protein. A typical column profile is shown in Fig. 19.

The minactivin produced by this method had a specific activity of 63 units/mg and was recovered in a yield of 35%. This represents a 10 fold increase in specific activity.

### Purification Example 4: Preparative Nondenaturing Gel Electrophoresis

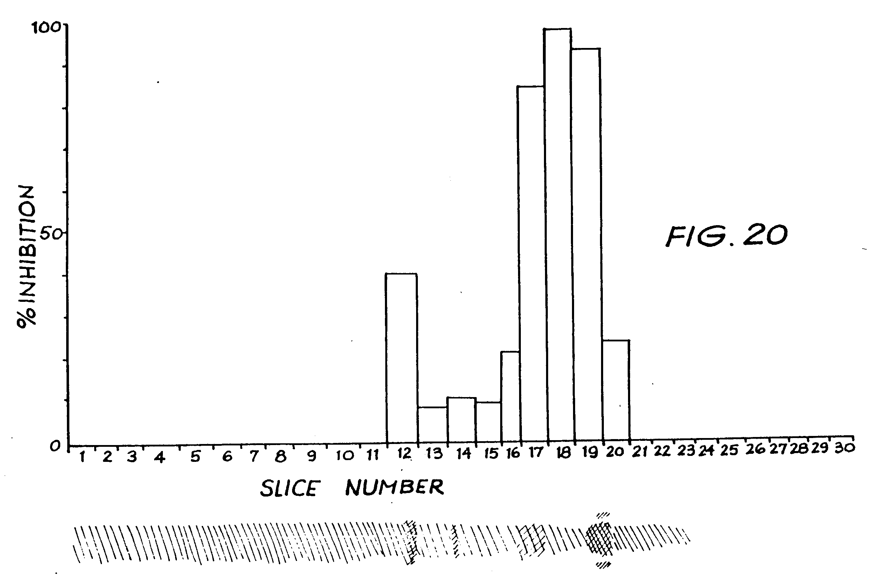

Cell free supernatants were processed through steps (a), (b) and (c) as described in Purification Example 2. Minactivin (3.5 units, specific activity 25 u/mg) and appropriate protein standards were mixed with an equal volume of sample buffer before electrophoresis. The sample buffer consisted of 0.025% bromophenol blue, 0.025% xylene-cyanol, 50 mM Tris HCl, pH 7.5, 18% sucrose and 1% Triton X100. The gel was composed of a 5-15% linear bis-acrylamide gradient and the gel system used was basically that described by Laemmli (26) without the addition of sodium dodecyl-sulphate. 0.15% Triton X100 was substituted for SDS in the running buffer reservoirs only. The sample was applied to the gel and electrophoresed using a constant current of 100 mA. The temperature was maintained below 25°C using a water jacketed cooling system. The gel was removed when the tracking dye of lower mobility, xylene-cyanol, travelled the length of the gel. The gel was cut into sections (approximately 0.5 x 1 cm per track), 150 ul of 50 mM glycine, pH 7.8, was added, and the sample sonicated at 4°C using a Branson Sonifier cell disrupter for 10-20 seconds at a sonic power of 20. The protein was eluted from the gel sections overnight with constant agitation. The gel was then pelleted by centrifugation of the sample for 10 minutes at 14,930 xg, and the supernatant removed for assay of minactivin activity. Duplicate samples were run to allow the protein content to be visualized by silver stain (27) of the appropriate gel track. The results are given in Fig. 20.

The recovery of minactivin from the gel was high: 64% of the initial minactivin activity was recovered after one extraction. An additional 13% was recovered following a second extraction by the same method. The silver stained protein profile shows that the minactivin activity is well separated from a major protein contaminant in the preparation. The specific activity of the minactivin obtained was 2323 units/mg which represents 93 fold purification of minactivin in this step.

### Purification Example 5: Blue-Agarose Chromatography

Cell free supernatants were processed through step (a) and (b) described in Purification Example 2. Ten ml of the concentrated supernatant (600U; 20.0mg; specific activity 30.0) was applied to a 3.2 cm x 2.8 cm column of Blue-Agarose equilibrated in 100 mM Na Phosphate, pH 7.0. The column was washed with the same buffer and 8.1 ml fractions collected. Fractions were pooled in groups of seven, dialysed overnight at 4°C against 50 mM glycine, pH 7.8, and then assayed for protein content and minactivin activity. Minactivin was found to elute unretarded from this column and was quantitatively recovered with a four fold increase in specific activity, yielding a value of 120 units/mg.

### Purification Example 6: DEAE-Sephacel Chromatography using Salt Gradient Elution

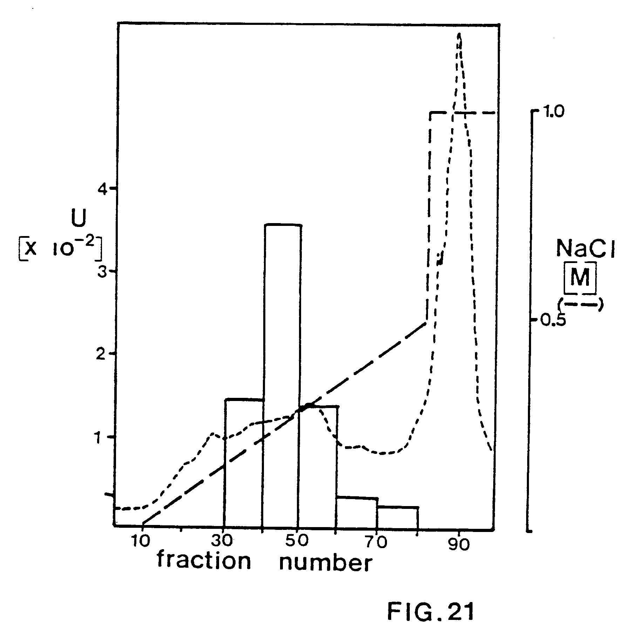

Cell free supernatants were processed through steps (a) and (b) described in Purification Example 2. Ten ml of the concentrated supernatant (640U; 20.0 mg: specific activity 32.0 U/mg) was applied to a 2.6 cm x 6 cm column of DEAE-Sephacel equilibrated with 50 mM glycine, pH 7.8. The column was washed with 50 ml of 50 mM glycine, pH 7.8 and then a 500 ml linear gradient of NaCl from 0 to 0.5 M in the same buffer applied. Upon completion of the gradient the column was washed with a further 90 ml of 50 mM glycine, pH 7.8 containing 1 M NaCl. Fractions of 6 ml were collected. Pools of 10 fractions were made, dialysed against 50 mM glycine, pH 7.8 overnight at 4°C and assayed for minactivin activity and protein content. The results are shown in Fig. 21. The minactivin eluted at 0.24 M NaCl and the peak pools gave 79% recovery with a specific activity of 216 U/mg. This represents a 6.75 fold purification of minactivin.

### Purification Example 7: DEAE-Sephacel Chromatography using pH Gradient Elution

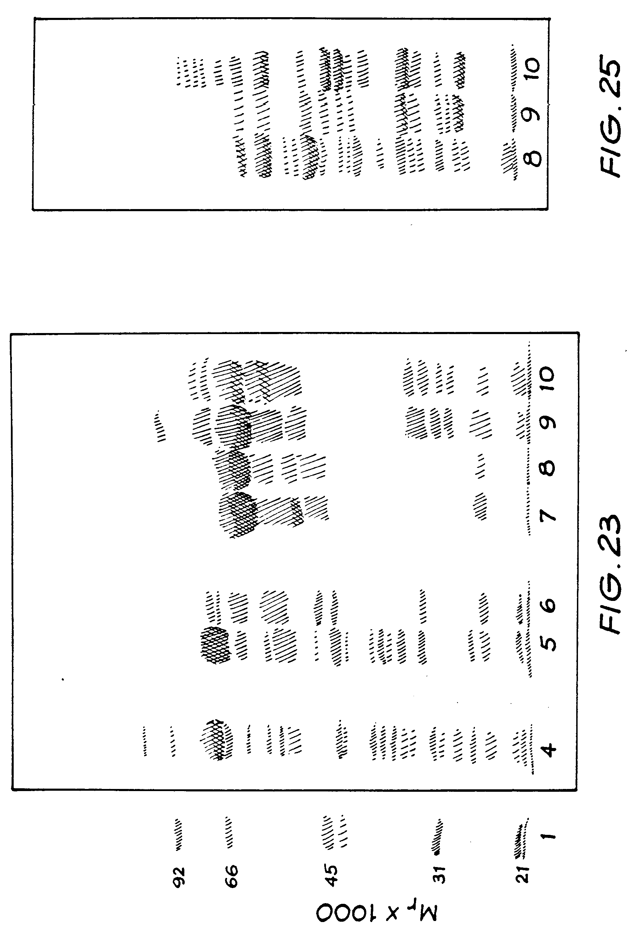

Cell free supernatants were processed through steps (a) and (b) as described in Purification Example 2. Ten ml of the concentrated media (400 U; 20 mg; specific activity 20.0 U/mg) was applied to a 2.6 cm x 6 cm column of DEAE-Sephacel equilibrated in 20 mM NH₄Ac, pH 6.9. The column was washed with 30 ml of 20 mM NH₄Ac, pH 6.9 and then a 500 ml gradient from 20 mM NH₄Ac to 20 mM acetic acid was applied to the column to elute the minactivin. Following completion of the gradient the column was washed with 20 mM acetic acid until the absorbance at 275 nm returned to the baseline. Fractions of 5.5 ml were collected. The fractions were pooled in groups of ten and the pH adjusted to between 7 and 8 with NaOH. 5.0 ml aliquots of each pool were dialysed overnight against 50 mM glycine, pH 7.8 at 4°C and then assayed for minactivin and protein content. The results are shown in Fig. 22. The peak pool of minactivin activity eluted at pH 5.3 and represented 74% of the original material applied to the column. This material had a specific activity of 1966 U/mg which represents a 98 fold purification. An aliquot equivalent to 70 ug of protein was removed from each pool and analysed by SDS-PAGE. The results are shown in Fig. 23.

### Purification Example 8: Hydroxylapatite Chromatography

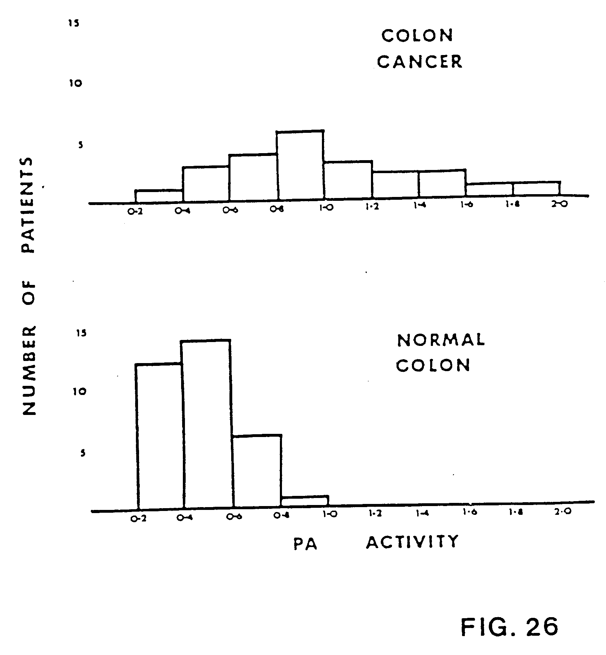

Cell free supernatants were processed through steps (a) and (b) as described in Purification Example 2. Ten ml of the concentrated supernatant (520 U; 16.6 mg; specific activity 31.0 U/mg) was applied to a hydroxylapatite column (2.6 cm x 4.5 cm) equilibrated in 50 mM glycine, pH 7.8. The column was washed with 50 mM glycine, pH 7.8 and then a 500 ml gradient from 50 mM glycine, pH 7.8 to 0.5 M sodium phosphate, pH 7.0 was applied. The fractions of 5.75 ml were pooled in groups of 10 and aliquots dialysed overnight at 4°C. These aliquots were then assayed for minactivin activity and protein content. Fig. 24 shows that the minactivin eluted at the very end of the gradient and the peak tubes contained 50% of the minactivin applied to the column. This material had a specific activity of 651 U/mg and represents a 21 fold purification. 70 ug aliquots of the minactivin containing fraction were analysed by SDS-PAGE and these are shown in Fig. 25.

### EXAMPLE 3

### EFFECT OF MINACTIVIN ON TUMOUR TISSUE

This example gives the details of the methods used followed by a description of the effect of minactivin on colon tumour cells.

### Methods

### 1. Tissue Samples and Homogenisation

Samples of human colons were obtained immediately after surgical resection. The colonic mucosa was dissected free from muscularis mucosa and washed with Hank's solution. Macroscopically normal mucosa and frank carcinoma were sampled from each colon and stored frozen. Portions of thawed material were later weighed and gently hand homogenised with 50 mM glycine buffer pH 7.8 containing 0.5% Triton X100, using 10 ul of buffer per mg (wet weight) of tissue.

### 2. Colorimetric Assays of Plasminogen Activators

The plasminogen activator content of tissue homogenates and cell culture supernatants were quantified by the assay method of Coleman and Green (12) as described in Example 1. Homogenates prepared as above were diluted 1:10 with homogenising buffer prior to assay.

### 3. SDS-PAGE and Fibrin Overlay Zymogram

The method of Granelli-Piperno and Reich (13) was used for this separation technique as described in Example 1. The tissue homogenates were applied to the non-reducing 11% acrylamide gel either (a) without further treatment, (b) after incubation for 30 min at 37° with affinity purified human plasminogen, (c) after incubation for 90 min at 23° with monocyte minactivin, or (d) after incubation first with plasminogen, and then with minactivin. Untreated samples of the above incubations (60 ul) were applied to the gel with SDS sample buffer (40 ul).

### 4. Radial Diffusion Assays

Radial diffusion assays were performed as described in Example 1. The proteases used for the incubations were kallikrein (1.5 ug), plasmin (150 ng), mouse urokinase (2.5 mPU), HPA66 (melanoma culture supernatant), human urokinase (12.5 mPU) and COLI 394 superantant (containing 1.3 mPU HPA52). These enzymes were incubated with excess minactivin for 90 min at 23°, then applied to the wells in the diffusion gel.

### 5. Culture of Colon Carcinoma Cells

The human colon carcinoma cell line COLO 394 (28) was cultured in RPMI-1640 supplemented with 10% foetal calf-serum and passaged twice a week.

For studies of the effect of minactivin and tumour cell culture enzyme, approx. 3 x 10⁶ cells/well were plated in 6-place multi-well plates (Linbro No. 76-058-05). After adherence overnight, the cells were washed and cultured with serum-free RPMI 1640 for 48 hours. Additions to the three experimental cultures were 1) human plasminogen (15 ug/ml, 2) plasminogen plus minactivin (equivalent to 1 PU of urokinase) or 3) plasminogen, minactivin and trasylol (18 ug/ml).

### 6. Minactivin Preparation

Minactivin used in these experiments was prepared from human blood monocyte culture supernatants as described in Purification Example 6. Adherent monocyte cultures on plastic dishes were cultured for 3 days with RPMI 1640 containing 0.05 ug/ml of muramyl depeptide.

### Results

### EFFECT OF MINACTIVIN ON TUMOUR TISSUE HOMOGENATES

### 1. Comparison of the Plasminogen Activator Content of Normal and Tumour Colon Tissues

### a) Colorimetric Assay

Diluted homogenates of colon mucosa were assayed for plasminogen activator by the method of Coleman and Green (12). This assay measures the total content of plasminogen activator enzyme (i.e. proenzyme and activate enzyme) because the plasminogen substrate used contains trace amounts of plasmin sufficient to activate proenzyme. Direct hydrolysis of the lysine thioester plasmin substrate by plasminogen-independent neutral proteases in the diluted homogenates did not contribute significantly to colour development. Both histologically normal areas of cancer-bearing colons, and frank colon cancer tissue were assayed for plasminogen activator activity as shown in Fig. 26.

The activity of normal tissue homogenates was tightly centred, with a mean value of 0.51 ± 0.16. While only a small minority of samples showed active urokinase bands of SDS-PAGE fibrin overlay (see below), the more sensitive colorimetric assay showed that all homogenates of normal tissue contained some plasminogen activator contributing to this assay. In the absence of added fibrin, it seems most likely this contribution was from low levels of urokinase-type plasminogen activator (HPA52) and not from the ubiquitous tissue-type plasminogen activator (HPA66)(29).

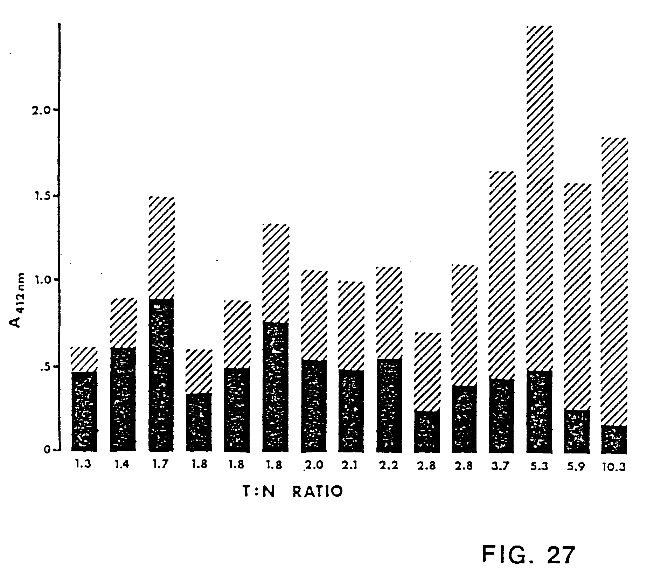

Tumour homogenates, however, showed a wide spectrum of plasminogen activator activity, covering a ten fold absorbance range. The mean absorbance was 1.15 ± 0.56, more than double that of the mean for normal tissue homogenates. This ratio however included all material sampled, and if instead the tumour samples were paired with the corresponding samples of normal tissue from each colon (Fig. 27), the mean ratio increased to over three, with some pairs having a ratio of greater than five.

### b) SDS-PAGE and Fibrin Overlay

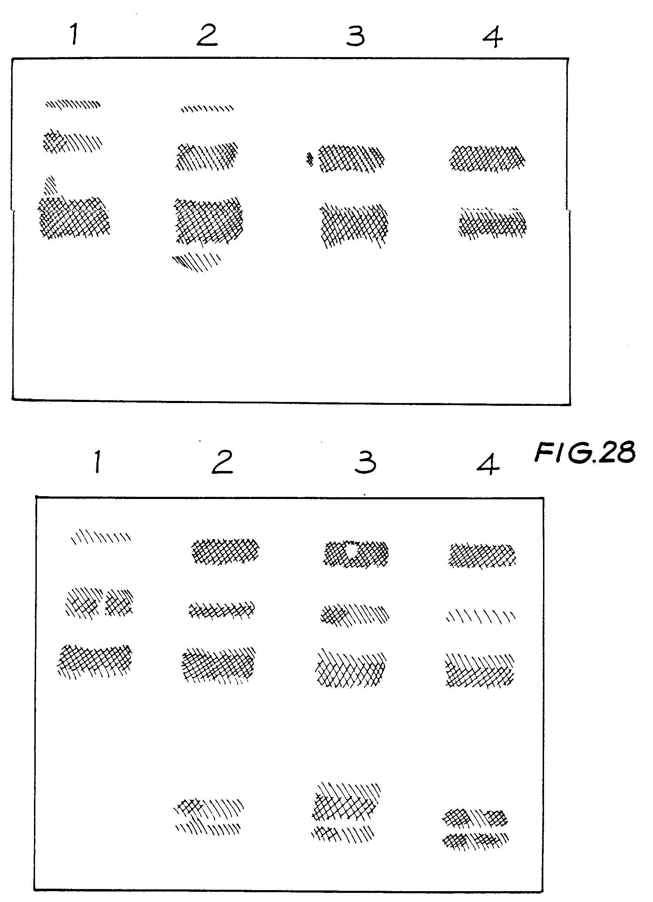

Homogenates of colon mucosa from histologically normal areas of tumour-bearing colons typically produced only one major band of lysis on fibrin-agarose zymograms after SDS-PAGE which corresponded to the tissue-type plasminogen activator (HPA66) of human melanoma culture supernatant (Fig. 28A). Minor bands were present at molecular weights of approximately 96,000 and 110,000. However, some homogenates of normal tissue also produced a lysis band corresponding to the high molecular weight form of urokinase. i.e. HPA52 (see below). If undiluted homogenates of typically normal colon were incubated with human plasminogen in the assay buffer, some activation of plasminogen occurred and a plasmin band appeared on the zymogram at Mᵣ 85,000 (Fig. 28A, lane 2). HPA66 activity progressively disappeared when samples were removed following a 2 hour incubation with plasminogen, the minor bands at 96,000 and 110,000 also disappeared rapidly.

Homogenates of cancer tissue typically produced a major band of HPA52, with a second band of HPA66 of varying relative intensity (sometines almost absent) (Fig. 28B). The other minor bands noted above were usually very weak or absent. Pre-incubation with plasminogen resulted in the gradual disappearance of HPA66 after 2 hrs at 37° (Fig. 28B, lanes 2-4). The plasmin produced (lysis at Mᵣ 85,000) then effected the conversion of HPA52 to its active cleavage products with Mᵣ of approx. 33-36,000.

### 2. Interaction of Minactivin with Urokinase-type Plasminogen Activators in Colon Mucosa Homogenates

Treatment of normal colon tissue homogenates with minactivin before electrophoresis had no effect on the lysis band produced by the tissue-type plasminogen activator, HPA66, but led to the loss of nearly all of the minor activity band of HPA52 (Fig. 29A).

Preincubation of the homogenate with plasminogen produced plasmin, which diminished the HPA52 band and led to the appearance of trace bands representing its active degradation products of Mᵣ 33-36,000. When the preincubation with plasminogen was followed by treatment with minactivin, the HPA52 band and the trace bands at 33-36,000 were abolished.

Tumour tissue homogenates produced prominent HPA52 lysis bands which were diminished by incubation with minactivin (Fig. 29B). Pretreatment with plasminogen before electrophoresis produced plasmin and this in turn converted the HPA52 to HPA33-36 as observed previously for the normal tissue homogenate. Thus, although minactivin had no effect on HPA66, the enzymes in homogenates of both tumour colon tissues and those normal colon tissues which produced bands of Mᵣ 52,000 were similarly affected by incubation with human monocyte minactivin. If the homogenates were treated initially with plasminogen and then with minactivin, the HPA52 band normally seen after electrophoresis essentially disappeared and the bands representing HPA33-36 were abolished (Fig. 29B). Thus, it is apparent that inactivation of HPA52 by minactivin following plasminogen treatment is considerably more effective than that observed in untreated homogenates. This suggests that the predominant form of the HPA52 occurring in tissues is in fact the proenzyme, and that reaction with minactivin is dependent on, its conversion to active enzyme, probably by means of plasmin.

### 3. Specificity of Minactivin with Respect to the Proenzyme Form of Urokinase-type Plasminogen Activator Colorimetric Assays

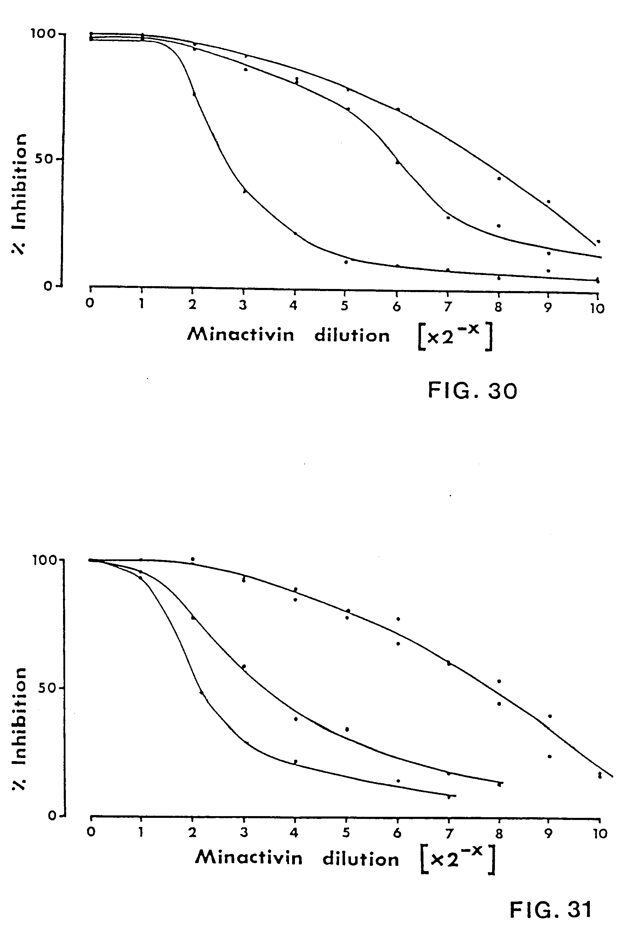

The stoichiometric relationship between minactivin and urokinase holds over a wide range of urokinase concentrations, from 10 mPU to 1000 mPU (Fig. 30). However, titration of the minactivin inhibition of plasminogen activator in dilute homogenates of colon mucosa did not exhibit this characteristic feature (Fig. 31). Indeed, more minactivin was required to inhibit the low level of activity of HPA 52 in normal tissue than to inhibit the much enhanced activity in tumour tissue homogenates. This is consistent with the presence of the proenzyme form of HPA52 in the tissue homogenates.

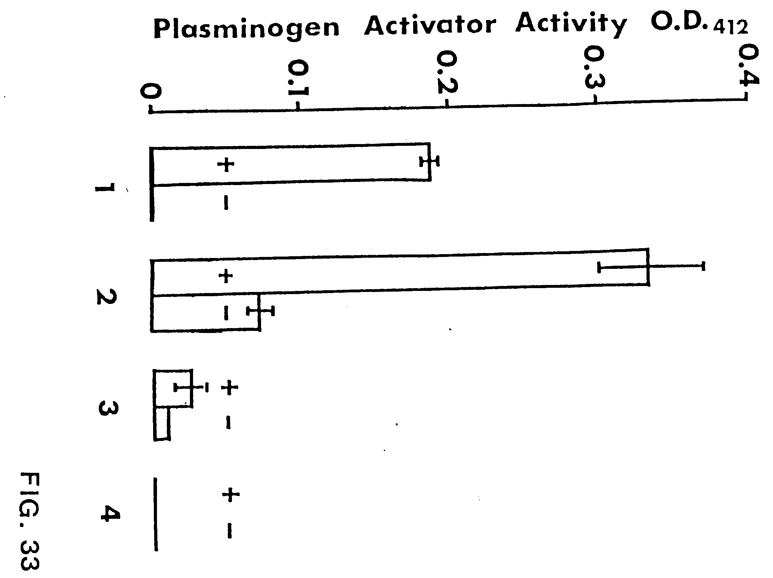

The presence of this HPA52 proenzyme can be demonstrated using serum free supernatants from cultures of the transformed colon tumour cell line COLO394. The plasminogen activator secreted by this cell line consists principally of HPA52 with some other higher Mᵣ bands, presenting a very similar pattern to the tumour tissue homogenates above (Fig. 32). When plasminogen was included in the culture media, some plasmin was produced (shown by colorimetric assay, Fig. 33) which precipitated the conversion of some HPA52 to HPA36. If minactivin and plasminogen were added to the media, all the plasminogen activator produced during the culture was inactivated, as shown by both SDS-PAGE and fibrin overlay development (Fig. 32) and colorimetric assay (Fig 33). However, if trasylol, a rapid and potent inhibitor of plasmin, was added to cultures together with plasminogan and minctivin, no inactivation occurred (Fig. 32). Since trasylol does not directly affect the reaction between minactivin and urokinase, these results showed that the HPA52 produced by COLO 394 was in the proenzyme form, requiring plasmin for expression of activity and for reaction with minactivin. In the SDS-PAGE fibrin overlay system, activation occured due to the traces of plasmin present in the plasminogen substrate. This did not occur when plasmin was inhibited by trasylol.

These results demonstrate that several major human carcinomas produce significantly greater amounts of urokinase-type plasminogen activator than that occurring in adjacent uninvolved tissue. Minactivin is capable of at least partially inhibiting HPA52, a human urokinase-type plasminogen activator, when added directly to tissue homogenates. This inactivation is specific for the urokinase-type plasminogen activator, HPA66. It is further established that minactivin does not affect the proenzyme form of HPA52, but that protease activity (in this case provided by plasmin) is required for the conversion of HPA52 to an active form before it is available to react with minactivin.

### REFERENCES

1. Hume, D., Gordon, S. (1983). J. Cell Physiol. 117: 189
2. Golder, J.P., Doe, W.F. 91983). Gastroenterology 84: 795.
3. Doe, W.F., Henson, P.M. (1978). J. Exp. Med. 148: 544.
4. Sundstrom, C., Nilsson, K. (1976). Int. J. Cancer 17: 565.
5. Gallagher, R.S., Collins, S., Trujillo, J. (1979). Blood 54: 713.
6. Bradley, T.R., Pilkington, G.R., Garson, M., Hogdson, G.S., Kraft, N. (1982). Br. J. Haematol. 51: 595.
7. Rabin, H., Hopkins R.F., Ruscetti, F.W., Neubauer, R.H., Brown. R.L. kawakami, T.G. (1981). J. Immunol. 127: 1852.
8. Lozzio, C.B., Lozzio, B.B. (1975). Blood 45 : 321, 1975.
9. Koerten, H.K., Ploem, J.S., Daems, W.R. (1980). Exp. Cell Res. 128; 470.
10. Yam, L.T., Li. C.Y., Crosby, W.H. (1971). Am. J. Clin. Path. 55: 282.
11. Ornstein. L., Ansley, H., Saunders, A. (1976). Blood Cells 2: 557.
12. Coleman, P.L., Green, G.D.J. (1981). Ann. NY Acad Sci 370: 617.
13. Unkeless, J., Dano, K., Kellermann, G., Reich, E. (1974). J. Biol. Chem. 249: 4295.
14. Granelli-Piperno, A. & Reich, E. (1978). J. Exp. Med. 148: 223-234
15. Crutchely, D.J., Conanan, L.B. & Maynard, J.R. (19821). Ann. N.Y. Acad. Sci. 370: 609-616.
16. Hoylaerts, M., Rijken, D.C., Lijnen, H.R. & Collen, D. (1982) J. Biol. Chem. 257: 2912-2919.
17. Barlow, G.H., Francis, C.W. & Narder, V.J. (1981). Thromb. Res. 23: 541-547.
18. Loskutoff, D.J. & Edgington, T.S. (1981). J. Biol. Chem. 246: 4142-4145
19. Aggeler, J., Risch, J. & Werb, A. (1981). Biochem. Biophys. Acta. 675: 62-68.
20. Ellouz, F., Adam, A., Ciorbaru, R. & Lederer, R., (1974). Biochem. Biophys. Res. Commun. 59: 1317-1325.
21. Reich, E. (1978). In Molecular Basis of Biological Degradative Processes (Berlin, R.D., Herman, L.L., Lepow, I.H. & Tranzer, J.M. Eds.) Academic Press, New York, p.155-159.
23. Holmberg, L., Lecander, I., Persson, B., Astedt, B. (1978). Biochem. Biophy. Acta. 544: 128-137.
24. Lowry, O.H., Rosebrough, N.J., Farr, A.L., Randall, R.J. (1951). J. Biol. Chem. 193: 265.
25. Bradford, M.M. (1976). Anal. Biochem. 72: 248-254.
26. Laemmlli, U.K. (1970). Nature 227, 680-685.
27. Merril, C.R., Goldman, D., Sedman, S.A., Ebert, M.H. (1981). Science 211: 1437.
28. Quinn, L.A., Moore, G.E., Morgan, R.T. and Woods, L.K. (1979). Cancer Res. 39: 4914-4924.
29. Rijken, D.C., Hoylaerts, M., Collen, D. (1982). J. Biol. Chem. 257: 2920-2925.
30. Camiolo, S.M., Markus, G., Evers, J.L., Hobika, G.H., De Pasquale, J.L., Beckley, S., Grimaldi, J.P. (1981). Int. J. Cancer 97: 191-198.
31. Corasanti, J.G., Celik, C., Camiolo, S.M., Mittelman, A., Evers, J.L., Barbasch, A., Hobika, G.H., Markus, G. (1980). J. Nat. Cancer Inst. 65: 345-351.
32. Evers, J.E., Patel, J., Madeja, J.M., Schneider, S.L., Hobika, G.H., Camiolo, S.M., Markus, G. (1982). Cancer Res. 42: 219-226.
33. Markus, G., Takita, H., Camiolo, S.M., Corasanti, J.G., Evers, J.L., Hobika, G.H. (1980). Cancer Res. 40: 841-848.
34. Camiolo, S.M., Markus, G., Englander, L.S., Siuta, M.R., HObika, G.H., Kohga, S. (1984). Cancer Res. 44: 311-318.
35. Marcus, G., Camiolo, S.M., Kohga, S., Madeja, J.M., Mittelman, A. (1983). Cancer Res. 43: 5517-5525.
36. Strassburger, W., Wollmer, A., Pitts, J.E., Glover, I.D., Tickle, I.J., Blundell, T.L., Steffens, G.J., Gunzler, W.A., Otting, F., Flohe, L. (1983). FEBS Letters 157: 219-223.
37. Kristensen, P., Larsson, L-I, Nielsen, L.S., Grondahl-Hansen, J., Andreasen, P.A., Dano, K. (1984). FEBS Letters 168: 33-37.
38. Duffy, M.J., O'Grady, P. (1984). Eur. J. Cancer Clin. Oncol. 20: 577-582.
39. Blumberg, P.M., Robbins, P.W. (1975). Cell 6: 137-147.
40. Sutherland, D.J.A. (1980). J.N.C.I. 64: 3-7
41. Ossowski, L., Quigley, J.P., Reich, E. (1975) in Proteases and Biological Control (Reich, E., Rifkin, D.B., Shaw, E. eds.) New York, Cold Spring Harbor Laboratory, p.901-913.
42. Day, E.D., Planinsek, J.A., Pressman, D. (1959). J.N.C.I. 22: 413-426.
43. O'Grady, R.L., Upfold, L.I., Stephens, R.W. (1981). Int. J. Cancer 28: 509-515.
44. Skriver, L., Larsson, L.-I., Kielberg, V., Nielsen, L.S., Andersen, P.B., Kristensen, P., Dano, K. (1984). J. of Cell Biol. 99: 752-757.
45. Taylor, C.R. (1978). Arch. Pathol. Lab. Med. 102: 113-121.
46. Soule, H.R., Linder E., Edgington, T.S. (1983). Proc. Natl.. Acad. Sci. 80: 1332-1336.
47. Ryman, B.E., Barratt, G.M., Begent, R.H.J. (1983). Biol. Cell 47: 71-80.
48. Richardson, V.J., Ryman, B.E., Jewkes, R.F., Jeyasingh, K., Tattersasll, M.H.N., Newlands, E.S., Kaye, S.B. (1979). B.J. Cancer 40: 35-43.
49. Osborne, M.P., Richardson, V.J., Jeyasingh, K., Ryman, B.E. (1982). Int. J. Nucl. Med. Biol. 9: 47-51.
50. Begent, RH.J., Keep, P.A., Green, A.J., Searle, F., Bagshawe, K.D., Jewkes, R.F., Jones, B.E., Barratt, G.M., Ryman, B.E. (1982). Lancet 739-742.

## Claims

1. The protein minactivin:
(a) having an apparent molecular size of 60,000-70,000 by gel filtration chromatography, identical to a serum albumin standard;
(b) capable of specifically inhibiting human urokinase-type plasminogen activator but not tissue-type plasminogen activator in the presence of fibrin;
(c) being heat labile at temperatures above 56°C;
(d) being stable to freezing at -20°C and thawing;
(e) being stable in a pH range of 5 to 9 at 4°C;
(f) having a pI between 5.0 and 7.8;
(g) being capable of forming a detergent resistant complex with human urokinase or human urokinase-type plasminogen activator;
(h) producing at least one band with a Mᵣ of 32kD, 39-48kD or 60 kD on non-reducing SDS-PAGE which band(s) disappear(s) when the protein is incubated with urokinase; and
(i) being purified to a specific activity of at least about 8,900 U/mg.

2. Minactivin according to claim 1 purified to the order of about 8,900 U/mg.

3. A process for the preparation of minactivin as defined in claim 1 or claim 2, which process comprises stimulating cells producing minactivin, as defined in claim 1 or 2, the cells being cultured in vitro; and recovering minactivin from the culture supernatant by hydrophobic interaction chromatography followed by affinity chromatography or preparative non-denaturing gel electrophoresis.

4. The process according to claim 3 wherein the minactivin-producing cells are chosen from monolayer cultures of human blood monocytes, minactivin-producing macrophages or transformed cells of monocytic origin.

5. A process for the production of minactivin, as defined in claim 1 or 2, which process comprises in vitro stimulation of cells to produce minactivin using bacterial lipopolysaccharide, dexamethasone or a phorbol ester and the subsequent recovery of minactivin from the culture supernatant by hydrophobic interaction chromatography followed by affinity chromatography or preparative non-denaturing gel electrophoresis.

6. A process according to claim 5 wherein the phorbol ester is phorbol myristate acetate.

7. A process according to any one of claims 3 to 6 wherein the hydrophobic interaction chromatography is phenyl Sepharose chromatography.

8. A process according to any one of claims 3 to 7 wherein the affinity chromatography is Cibacron Blue Sepharose chromatography.

9. A reagent for locating and defining the boundaries of tumours in histological specimens in vitro comprising minactivin, as defined in claim 1 or 2, in suitably labelled form.

10. A reagent according to claim 9 wherein the minactivin is labelled with a radioactive isotope.

11. A reagent according to claim 9 wherein the label is a chemical compound such as fluorescein.

12. A reagent according to claim 9 wherein the minactivin is labelled with an enzyme.

13. A method of locating and defining the boundaries of a tumour in a histological specimen which comprises applying suitably labelled minactivin, as defined in claim 1 or 2, to the specimen and subsequently imaging to determine the site of concentration of the label.

14. A reagent for use in the method defined by claim 13 comprising minactivin as defined in claim 1 or 2 labelled with technetium⁹⁹.

15. A method according to claim 13 wherein the tumours are solid tumours.

16. A method according to claim 13 wherein the tumours are metastatic cancers.

17. A method of monitoring chronic inflammation comprising the detection of minactivin in isolated samples of human body fluids and tissues using antibodies prepared against minactivin, as defined in claim 1 or 2.

18. An antibody preparation prepared against minactivin, as defined in claim 1 or 2, for use in the method of claim 17.

19. An antibody preparation according to claim 18 wherein said antibody is labelled with an enzyme, an isotope or another chemical reagent.

20. Minactivin, as defined in claim 1 or 2, for use in diagnostic or therapeutic medicine.

21. The use of minactivin, as defined in claim 1 or 2, in the preparation of an agent for monitoring chronic inflammation.

## Patentansprüche

1. Das Protein Minaktivin, das
(a) ein durch Gelfiltration bestimmtes Molekulargewicht von 60000-70000 aufweist, das identisch ist mit einem Serumalbumin-Standard;
(b) in der Lage ist, spezifisch den menschlichen Plasminogenaktivator vom Urokinase-Typ, aber nicht den Plasminogenaktivator vom Gewebetyp in Gegenwart von Fibrin zu hemmen;
(c) bei Temperaturen über 56°C hitzelabil ist;
(d) bei Einfrieren bei -20°C und Auftauen stabil ist;
(e) in einem pH-Bereich von 5 bis 9 bei 4°C stabil ist;
(f) einen pI-Wert zwischen 5,0 und 7,8 aufweist;
(g) in der Lage ist, mit menschlicher Urokinase oder mit dem menschlichen Plasminogenaktivator vom Urokinase-Typ einen Komplex zu bilden, der gegen Detergentien beständig ist;
(h) bei nichtreduzierender SDS-Polyacrylamid-Gelelektrophorese mindestens eine Bande mit einem Mᵣ von 32 kD, 39-48 kD oder 60 kD erzeugt, wobei die Bande oder Banden verschwinden, wenn das Protein mit Urokinase inkubiert wird;
(i) bis zu einer spezifischen Aktivität von mindestens etwa 8900 U/mg gereinigt ist.

2. Minaktivin nach Anspruch 1, das bis zu einer Größenordnung von etwa 8900 U/mg gereinigt ist.

3. Verfahren zur Herstellung von Minaktivin nach Anspruch 1 oder Anspruch 2, umfassend die Stimulation von Minaktivin nach Anspruch 1 oder 2 produzierenden Zellen, wobei die Zellen in vitro gezüchtet werden, und das Gewinnen von Minaktivin aus dem Kulturüberstand durch hydrophobe Wechselwirkungschromatographie, gefolgt von Affinitätschromatographie oder präparativer nicht-denaturierender Gelelektrophorese.

4. Verfahren nach Anspruch 3, in dem die Minaktivin produzierenden Zellen aus Einzelschichtkulturen menschlicher Blutmonocyten, Minaktivin produzierender Macrophagen oder transformierter Zellen monocytischen Ursprungs ausgewählt sind.

5. Verfahren zur Herstellung von Minaktivin nach Anspruch 1 oder 2, umfassend die in vitro-Stimulation von Zellen zur Minaktivinproduktion unter Verwendung von bakteriellem Lipopolysaccharid, Dexamethason oder einem Phorbolester und anschließendes Gewinnen von Minaktivin aus dem Kulturüberstand durch hydrophobe Wechselwirkungschromatographie, gefolgt von Affinitätschromatographie oder präparativer nicht-denaturierender Gelelektrophorese.

6. Verfahren nach Anspruch 5, in dem der Phorbolester Phorbolmyristatacetat ist.

7. Verfahren nach einem der Ansprüche 3 bis 6, in dem die hydrophobe Wechselwirkungschromatographie Phenylsepharose-Chromatographie ist.

8. Verfahren nach einem der Ansprüche 3 bis 6, in dem die Affinitätschromatographie Cibacronblau-Sepharose-Chromatographie ist.

9. Reagens zur in vitro-Lokalisierung und Definition der Grenzen von Tumoren in histologischen Proben, umfassend Minaktivin nach Anspruch 1 oder 2 in geeignet markierter Form.

10. Reagens nach Anspruch 9, in dem das Minaktivin mit einem radioaktiven Isotop markiert ist.

11. Reagens nach Anspruch 9, in dem die Markierung eine chemische Verbindung wie Fluorescein ist.

12. Reagens nach Anspruch 9, in dem das Minaktivin mit einem Enzym markiert ist.

13. Verfahren zur Lokalisierung und Definition der Grenzen von Tumoren in einer histologischen Probe, umfassend die Anwendung von geeignet markiertem Minaktivin nach Anspruch 1 oder 2 auf die Probe und anschließendes Erstellen einer Abbildung zur Bestimmung der Lage der Konzentration der Markierung.

14. Reagens zur Verwendung im Verfahren nach Anspruch 13, umfassend Minaktivin nach Anspruch 1 oder 2, das mit Technetium⁹⁹ markiert ist.

15. Verfahren nach Anspruch 13, wobei die Tumoren feste Tumoren sind.

16. Verfahren nach Anspruch 13, wobei die Tumoren Metastasen bildende Tumoren sind.

17. Verfahren zur Überwachung chronischer Entzündungen, umfassend die Bestimmung von Minaktivin in isolierten Proben menschlicher Körperflüssigkeiten und -gewebe unter Verwendung von Antikörpern gegen Minaktivin nach Anspruch 1 oder 2.

18. Antikörperpräparat gegen Minaktivin nach Anspruch 1 oder 2 zur Verwendung im Verfahren nach Anspruch 17.

19. Antikörperpräparat nach Anspruch 18, in dem der Antikörper mit einem Enzym, einem Isotop oder einem anderen chemischen Reagens markiert ist.

20. Minaktivin nach Anspruch 1 oder 2 zur Verwendung in der diagnostischen oder therapeutischen Medizin.

21. Verwendung von Minaktivin nach Anspruch 1 oder 2 zur Herstellung eines Mittels zur Überwachung chronischer Entzündungen.

## Revendications

1. Protéine minactivin :
(a) ayant une taille moléculaire apparente de 60 000 à 70 000 par chromatographie de filtration sur gel, identique à un standard de sérum albumine;
(b) capable d'inhiber spécifiquement un activateur de plasminogène de type urokinase humain, mais pas un activateur de plasminogène de type tissulaire en présence de fibrine;
(c) étant labile à la chaleur à des températures supérieures à 56°C;
(d) étant stable à la congélation à -20°C et à la décongélation;
(e) étant stable dans un domaine de pH de 5 à 9 à 4°C;
(f) ayant un pI entre 5,0 et 7,8;
(g) étant capable de former un complexe résistant aux détergents avec de l'urokinase humaine ou d'un activateur de plasminogène de type urokinase humain;
(h) produisant au moins une bande d'un Mᵣ de 32 kD, 39-48 kD ou 60 kD par électrophorèse sur gel de polyacrylamide-SDS non réducteur, laquelle ou lesquelles bandes disparaissent quand la protéine est incubée avec de l'urokinase, et
(i) étant purifiée à une activité spécifique d'au moins environ 8900 U/mg.

2. Minactivin suivant la revendication 1, purifiée jusqu'à un ordre d'environ 8900 U/mg.

3. Procédé pour la préparation de minactivin suivant la revendication 1 ou la revendication 2, lequel procédé comprend la stimulation de cellules produisant de la minactivin, comme défini dans la revendication 1 ou 2, les cellules étant mises en culture in vitro; et la récupération de la minactivin du surnageant de culture par chromatographie à interactions hydrophobes suivie par chromatographie d'affinité ou électrophorèse préparative sur gel non dénaturant.

4. Procédé suivant la revendication 3, où les cellules produisant la minactivin sont choisies parmi des cultures monocouches de monocytes de sang humain, de macrophages produisant de la minactivin ou des cellules transformées d'origine monocytique.

5. Procédé pour la production de minactivin, comme défini dans la revendication 1 ou 2, lequel procédé comprend la stimulation in vitro des cellules pour produire de la minactivin au moyen de lipopolysaccharide bactérien, de dexaméthasone ou d'un ester de phorbol et la recupération subséquente de minactivin des surnageants de culture par chromatographie à interactions hydrophobes suivie par chromatographie d'affinité ou électrophorèse préparative sur gel non dénaturant.

6. Procédé suivant la revendication 5, où l'ester de phorbol est l'acétate de myristate de phorbol.

7. Procédé suivant l'une quelconque des revendications 3 à 6, où la chromatographie à interactions hydrophobes est une chromatographie sur du phénylSépharose.

8. Procédé suivant l'une quelconque des revendications 3 à 7, où la chromatographie d'affinité est une chromatographie sur Sépharose Bleue Cibacron.

9. Réactif pour localiser et définir les limites de tumeurs dans des spécimens histologiques in vitro comprenant de la minactivin, comme défini dans, les revendications 1 ou 2, sous une forme marquée appropriée.

10. Réactif suivant la revendication 9, où la minactivin est marquée avec un isotope radioactif.

11. Réactif suivant la revendication 9, où le marqueur est un composé chimique tel que la fluorescéine.

12. Réactif suivant la revendication 9, où la minactivin est marquée par une enzyme.

13. Procédé de localisation et de définition des limites d'une tumeur dans un spécimen histologique qui comprend l'application de minactivin marquée de manière appropriée, comme défini dans la revendication 1 ou 2, au spécimen et ensuite imagerie pour déterminer le site de concentration du marqueur.

14. Réactif à utiliser dans le procédé défini par la revendication 13, comprenant de la minactivin suivant la revendication 1 ou 2, marquée au technétium⁹⁹,

15. Procédé suivant la revendication 13, où les tumeurs sont des tumeurs solides.

16. Procédé suivant la revendication 13, où les tumeurs sont des cancers métastatiques.

17. Procédé de contrôle d'inflammations chroniques comprenant la détection de minactivin dans des échantillons isolés de fluides et de tissus du corps humain au moyen d'anticorps préparés contre la minactivin, comme défini dans la revendication 1 ou 2.

18. Préparation d'anticorps préparés contre la minactivin, comme défini dans la revendication 1 ou 2, pour l'utilisation dans le procédé de la revendication 17.

19. Préparation d'anticorps suivant la revendication 18, où l'anticorps est marqué par une enzyme, un isotope ou un autre réactif chimique.

20. Minactivin, comme défini dans la revendication 1 ou 2, pour l'utilisation dans la médecine diagnostique ou thérapeutique.

21. Utilisation de minactivin, comme défini dans la revendication 1 ou 2, dans la préparation d'un agent pour le contrôle d'inflammations chroniques.
